# EUROPEAN PATENT APPLICATION

(11) **EP 4 400 032 A1**
(43) Date of publication of application: **17.07.2024**
(21) Application number: 22866725.9
(22) Date of filing: 08.09.2022
(51) Int. Cl.: A61B 5/00

(54) **MONITORING METHOD AND MONITORING SYSTEM**

(30) Priority: 08.09.2021 CN 202111051456
(71) Applicant: Shenzhen Mindray Bio-Medical Electronics Co., Ltd., Shenzhen, Guangdong 518057 (CN)
(72) Inventor: LI, Xinsheng, Shenzhen, Guangdong 518057 (CN); FENG, Xiangying, Xi'an, Shanxi 710032 (CN); LIU, Zhonghua, Shenzhen, Guangdong 518057 (CN); LI, Liya, Shenzhen, Guangdong 518057 (CN); CEN, Jian, Shenzhen, Guangdong 518057 (CN); LUO, Hanyuan, Shenzhen, Guangdong 518057 (CN); LIU, Qiling, Shenzhen, Guangdong 518057 (CN)
(74) Representative: KIPA AB
(86) International application number: PCT/CN2022/117914
(87) International publication number: WO 2023/036264

(57) **Abstract**

A monitoring method and a monitoring system. The monitoring system comprises a bedside monitor (20), a mobile monitoring device (30), and a central station (10). The mobile monitoring device (30) receives patients' physiological parameter data detected by a spot measuring device (40), and transmits the data to the central station (10). The physiological parameter data of the spot measuring device (40) is transmitted to the central station (10), suitable for the scenario where a nurse detects basic physiological parameters of all patients. The mobile monitoring device (30) is paired with the bedside monitor (20) to continuously monitor the patients, and wirelessly transmits monitoring data obtained by the continuous monitoring to the central station (10) and/or the paired bedside monitor (20), suitable for patients in the critical period after surgery, patients with serious conditions in emergency observation, etc. When working in an interval monitoring mode, the mobile monitoring device (30) performs interval monitoring on the patients, and wirelessly transmits monitoring data obtained by the interval monitoring to the central station (10), suitable for patients who get out of bed for recovery after surgery, mild patients in emergency observation, etc. Hence, the monitoring system can satisfy the monitoring needs of different patients and a same patient at different stages.

## Description

### TECHNICAL FIELD

The disclosure relates to a medical field, and more particularly to a monitoring method and a monitoring system.

### BACKGROUND

Mobile monitoring is one of hot topics in clinical research in recent years. It mainly solves problems of low compliance and insufficient monitoring rate of patients who need monitoring, and the caused potential risks which cannot be measured in time, due to inconvenience of getting out of support, which inconvenience is caused by constraints of monitoring methods.

Mobile monitoring uses wearable sensors and wireless data transmission to monitor basic physiological parameters of patient, and is still capable of monitoring the patient without affecting its activities. The provided physiological parameters can satisfy personalized requirements in specific clinical scenarios. The wearable sensors and wireless data transmission manners are current focuses of various manufacturers, who have launched various product forms and diversified wireless transmission manners, which have been widely used in clinical applications.

However, due to the diversity and complexity of clinical requirements, simple mobile monitoring device cannot truly solve practical problems and be truly applied in clinical practice. Since the functions and performances of the mobile monitoring device cannot fully cover requirements of daily work scenarios, so that it is easy to cause problems such as increased workload of medical staff.

### SUMMARY

This disclosure mainly provides a monitoring method and a monitoring system, which are capable of satisfying different monitoring requirements.

An embodiment provides a monitoring system, including a bedside monitor, a mobile monitoring device and a central station; wherein:
the bedside monitor is configured to monitor a patient and pair with the mobile monitoring device;
the mobile monitoring device is worn on a body of the patient, so as to monitor the patient for obtaining monitoring data; wherein a working mode of the mobile monitoring device includes a continuous monitoring mode and an intermittent monitoring mode; wherein the mobile monitoring device is configured to:
   when working in the intermittent monitoring mode; perform intermittent monitoring on the patient, receive, in a first wireless transmission manner, physiological parameter data of the patient, which data is measured by a spot measurement device, and transmit, to the central station in a second wireless transmission manner, monitoring data which is obtained during the intermittent monitoring and the physiological parameter data which is measured by the spot measurement device;
   when working in the continuous monitoring mode; pair with the bedside monitor and jointly perform continuous monitoring on the patient with the bedside monitor, and transmit, to the central station and/or the paired bedside monitor in the second wireless transmission manner, monitoring data which is obtained during the continuous monitoring and physiological parameter data of the patient, which data is measured by a spot measurement device.

An embodiment provides a monitoring system, including:
a central station;
a mobile monitoring device, which is worn on a body of a patient, so as to monitor the patient for obtaining monitoring data; wherein a working mode of the mobile monitoring device includes a continuous monitoring mode and an intermittent monitoring mode; wherein the mobile monitoring device is configured to:
   when working in the intermittent monitoring mode; perform intermittent monitoring on the patient, receive, in a first wireless transmission manner, physiological parameter data of the patient, which data is measured by a spot measurement device, and transmit, to the central station in a second wireless transmission manner, monitoring data which is obtained during the intermittent monitoring and the physiological parameter data which is measured by the spot measurement device;
   when working in the continuous monitoring mode; perform continuous monitoring on the patient, and transmit, to the central station in the second wireless transmission manner, monitoring data which is obtained during the continuous monitoring and physiological parameter data of the patient, which data is measured by a spot measurement device.
   An embodiment provides a central station, which is applicable in a monitoring system, wherein the monitoring system includes: a mobile monitoring device which is in wireless communicative connection with the central station;
   the mobile monitoring device is worn on a body of a patient, so as to monitor the patient for obtaining monitoring data; wherein the mobile monitoring device is further configured to receive, in a first wireless transmission manner, physiological parameter data of the patient, which data is measured by a spot measurement device a working mode of the mobile monitoring device includes a continuous monitoring mode and an intermittent monitoring mode;
   the central station is configured to:
      receive, in a second wireless transmission manner, monitoring data which is obtained during intermittent monitoring and the physiological parameter data which is measured by the spot measurement device; wherein said monitoring data and the physiological parameter data are transmitted when the mobile monitoring device works in the intermittent monitoring mode;
      receive, in the second wireless transmission manner, monitoring data which is obtained during continuous monitoring and the physiological parameter data which is measured by the spot measurement device; wherein said monitoring data and the physiological parameter data are transmitted when the mobile monitoring device works in the continuous monitoring mode;
      display, according to the patient, the physiological parameter data which is measured by the spot measurement device; and
      process the received monitoring data, so as to obtain at least one of a physiological parameter value, a physiological parameter curve, and an alarm event, and display the physiological parameter value, the physiological parameter curve, and/or the alarm event according to the patient.

An embodiment provides a monitoring system, including:
a bedside monitor;
a mobile monitoring device, which is worn on a body of a patient, so as to monitor the patient for obtaining monitoring data; wherein a working mode of the mobile monitoring device includes a continuous monitoring mode and an intermittent monitoring mode; wherein the mobile monitoring device is configured to:
   when working in the intermittent monitoring mode; perform intermittent monitoring on the patient, receive, in a first wireless transmission manner, physiological parameter data of the patient, which data is measured by a spot measurement device, and transmit, to the bedside monitor in a second wireless transmission manner, monitoring data which is obtained during the intermittent monitoring and the physiological parameter data which is measured by the spot measurement device;
   when working in the continuous monitoring mode; pair with the bedside monitor and jointly perform continuous monitoring on the patient with the bedside monitor, and transmit, to the bedside monitor in the second wireless transmission manner, monitoring data which is obtained during the continuous monitoring and physiological parameter data of the patient, which data is measured by a spot measurement device.

An embodiment provides a monitoring system, including a mobile monitoring device and a spot measurement device; wherein:
the mobile monitoring device is worn on a body of a patient, so as to monitor the patient for obtaining first monitoring data; wherein the mobile monitoring device is in communicative connection with a target monitoring device;
the spot measurement device is configured to measure physiological parameter data of the patient;
the mobile monitoring device includes a processor, a first wireless communication unit and a second wireless communication unit; wherein the mobile monitoring device is configured to receive, in a first wireless transmission manner through the first wireless communication unit, the physiological parameter data of the patient, which data is measured by the spot measurement device, and to transmit, to a target monitoring device in a second wireless transmission manner through the second wireless communication unit, the first monitoring data and the received physiological parameter data of the patient, which data is measured by the spot measurement device; wherein the first wireless communication unit and the second wireless communication unit use different wireless communication protocols, and the first wireless transmission manner is different from the second wireless transmission manner.

An embodiment provides a monitoring system, including a mobile monitoring device and a spot measurement device; wherein:
the mobile monitoring device is worn on a body of a patient, so as to monitor the patient for obtaining first monitoring data; wherein the mobile monitoring device is in communicative connection with a target monitoring device;
the spot measurement device is configured to measure physiological parameter data of the patient;
the mobile monitoring device includes a processor, a first wireless communication unit and a second wireless communication unit; wherein the mobile monitoring device is configured to receive, in a first wireless transmission manner through the first wireless communication unit, the physiological parameter data of the patient, which data is measured by the spot measurement device, and to transmit, to a target monitoring device in a second wireless transmission manner through the second wireless communication unit, the first monitoring data and the received physiological parameter data of the patient, which data is measured by the spot measurement device; wherein the first wireless communication unit and the second wireless communication unit use different wireless communication protocols, and the first wireless transmission manner is different from the second wireless transmission manner.

An embodiment provides a spot measurement device which is applicable in a monitoring system; wherein the monitoring system includes a mobile monitoring device, which is in wireless communicative connection with the spot measurement device, and a target monitoring device, which is in wireless communicative connection with the mobile monitoring device; wherein the mobile monitoring device is worn on a body of a patient, so as to monitor the patient for obtaining first monitoring data; wherein the mobile monitoring device is in communicative connection with the target monitoring device so as to transmit the obtained first monitoring data to the target monitoring device, the spot measurement device is configured to measure physiological parameter data of the patient;
the spot measurement device includes a processor and a first wireless communication unit; wherein the spot measurement device is further configured to transmit, to the mobile monitoring device in a first wireless transmission manner through the first wireless communication unit, the physiological parameter data of the patient, which data is measured by the spot measurement device; so as to enable the mobile monitoring device to transmit, to a target monitor device in a second wireless transmission manner through a second wireless communication unit which is arranged at the mobile monitoring device, the received physiological parameter data of the patient, which data is measured by the spot measurement device; wherein the first wireless communication unit and the second wireless communication unit use different wireless communication protocols, and the first wireless transmission manner is different from the second wireless transmission manner.

An embodiment provides a monitoring method, including:
when a mobile monitoring device works in an intermittent monitoring mode; performing intermittent monitoring on a patient, receiving, in a first wireless transmission manner, physiological parameter data of the patient, which data is measured by a spot measurement device, and transmitting, to a central station in a second wireless transmission manner, monitoring data which is obtained during the intermittent monitoring and the physiological parameter data which is measured by the spot measurement device;
when a mobile monitoring device works in a continuous monitoring mode, pairing the mobile monitoring device with a bedside monitor and jointly performing continuous monitoring on a patient with the bedside monitor, and transmitting, to a central station and/or the paired bedside monitor in the second wireless transmission manner, monitoring data which is obtained during the continuous monitoring and physiological parameter data which is measured by a spot measurement device.

An embodiment provides a monitoring method, including:
when a mobile monitoring device works in an intermittent monitoring mode; performing intermittent monitoring on a patient, receiving, in a first wireless transmission manner, physiological parameter data of the patient, which data is measured by a spot measurement device, and transmitting, to a central station in a second wireless transmission manner, monitoring data which is obtained during the intermittent monitoring and the physiological parameter data which is measured by the spot measurement device;
when a mobile monitoring device works in a continuous monitoring mode, performing continuous monitoring on a patient, and transmitting, to the central station in the second wireless transmission manner, monitoring data which is obtained during the continuous monitoring and physiological parameter data which is measured by a spot measurement device.

An embodiment provides a monitoring method, including:
when a mobile monitoring device works in an intermittent monitoring mode; performing intermittent monitoring on a patient, receiving, in a first wireless transmission manner, physiological parameter data of the patient, which data is measured by a spot measurement device, and transmitting, to a bedside monitor in a second wireless transmission manner, monitoring data which is obtained during the intermittent monitoring and the physiological parameter data which is measured by the spot measurement device;
when a mobile monitoring device works in a continuous monitoring mode, pairing the mobile monitoring device with a bedside monitor and jointly performing continuous monitoring on a patient with the bedside monitor, and transmitting, to the paired bedside monitor in the second wireless transmission manner, monitoring data which is obtained during the continuous monitoring and physiological parameter data which is measured by a spot measurement device.

An embodiment provides a monitoring method, including:
when a mobile monitoring device works in an intermittent monitoring mode; performing intermittent monitoring on a patient, receiving, in a first wireless transmission manner, physiological parameter data of the patient, which data is measured by a spot measurement device, and transmitting, to a bedside monitor in a second wireless transmission manner, monitoring data which is obtained during the intermittent monitoring and the physiological parameter data which is measured by the spot measurement device;
when a mobile monitoring device works in a continuous monitoring mode, pairing the mobile monitoring device with a bedside monitor and jointly performing continuous monitoring on a patient with the bedside monitor, and transmitting, to the paired bedside monitor in the second wireless transmission manner, monitoring data which is obtained during the continuous monitoring and physiological parameter data which is measured by a spot measurement device.

The monitoring method and monitoring system according to the above embodiments include a bedside monitor, a mobile monitoring device and a central station. The mobile monitoring device receives, in a first wireless transmission manner, physiological parameter data of a patient, which data is measured by the spot measurement device; and transmits, to the central station in a second wireless transmission manner, the physiological parameter data. The physiological parameter data of the spot measurement device is transmitted to the central station, which is suitable for scenarios where nurses conduct basic physiological parameter testing on all patients. The mobile monitoring device pairs with the bedside monitor, so as to jointly perform a continuously monitoring on the patient, and wirelessly transmits the monitoring data obtained from the continuous monitoring to the central station and/or the paired bedside monitor, which is suitable for patients in postoperative dangerous period and patients under emergency observation. When the mobile monitoring device works in an intermittent monitoring mode, it performs intermittent monitoring on the patient and wirelessly transmits the monitoring data, which is obtained during the intermittent monitoring to the central station, which is suitable for patients who are getting out of support for recovery after surgery, mild patients under emergency observation, etc. It can be seen that the monitoring system of this disclosure can satisfy the monitoring requirements of different patients and the same patient at different stages.

### BRIEF DESCRIPTION OF THE DRAWINGS

FIG. 1 is a structural block diagram of an embodiment of a monitoring system provided by this disclosure.
FIG. 2 is a structural block diagram of an embodiment of a monitoring system provided by this disclosure.
FIG. 3 is a flow chart of an embodiment of a monitoring method for the monitoring system shown in FIG. 1.
FIG. 4 is a flow chart of an embodiment of a monitoring method for the monitoring system shown in FIG. 2.
FIG. 5 is a flow chart of an embodiment of a postoperative monitoring scenario in a monitoring system provided by this disclosure.
FIG. 6 is a flow chart of an embodiment of an emergency observation scenario in a monitoring system provided by this disclosure.
FIG. 7 is a structural block diagram of an embodiment of a monitoring system provided by this disclosure.
FIG. 8 is a structural block diagram of an embodiment of a monitoring system provided by this disclosure.
FIG. 9 is a structural block diagram of an embodiment of a monitoring system provided by this disclosure.

### DESCRIPTION OF THE PREFERRED EMBODIMENTS

This disclosure is further described in detail below through specific embodiments in combination with the accompanying drawings. In different embodiments, similar elements adopt associated similar reference numbers. In the following embodiments, many details are described in order to make this disclosure better understood. However, one skilled in the art can easily recognize that some of the features can be omitted in different cases, or can be replaced by other elements, materials and methods. In some cases, some operations related to this disclosure are not shown or described in the description, in order to avoid the core part of this disclosure being inundated by too many descriptions. For one skilled in the art, it is not necessary to describe these related operations in detail, as they can fully understand the relevant operations according to the description in this disclosure and the general technical knowledge in the art.

In addition, the features, operations, or characteristics described in the specification may be combined in any appropriate manner to form various embodiments. At the same time, the steps or actions in the method description can also be exchanged or adjusted sequentially in a manner obvious to one skilled in the art. Therefore, the various sequences in the description and the drawings are only for the purpose of clearly describing a certain embodiment, and do not mean that they are necessary sequences. Unless it is otherwise specified that one of the sequences must be followed.

The terms "first", "second", etc. in the specification are operable to distinguish different objects, rather than to describe a specific order. In addition, the terms "connection", and "linkage" mentioned in this disclosure include direct and indirect connection (linkage), unless otherwise specified.

This disclosure constructs a monitoring system involving mobile monitoring, with a mobile monitoring device as the core, combined with a variety of product forms and working manners, which can satisfy the diverse clinical requirements of sub-critical care departments, improve work efficiency, and finally build a system solution that can be widely used.

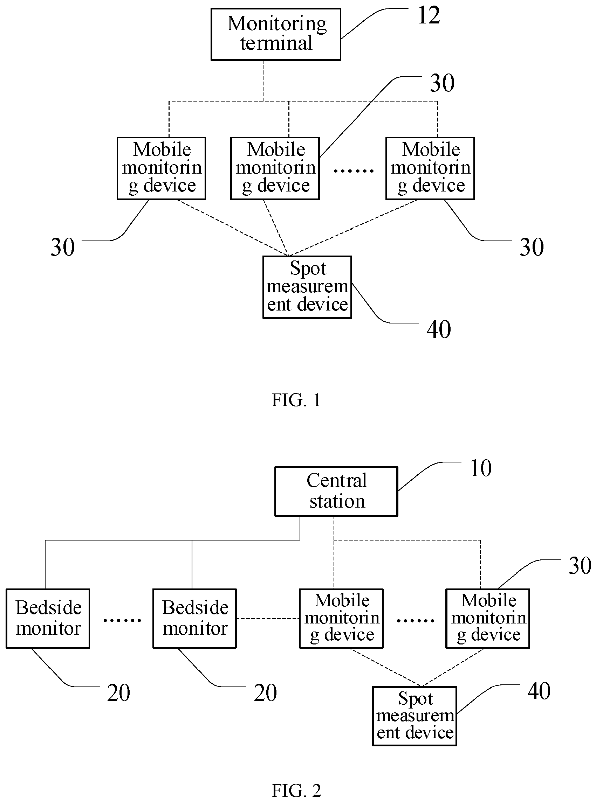

As shown in FIG. 1, the monitoring system provided by this disclosure includes a spot measurement device 40, a monitoring terminal 12 and multiple (two or more) mobile monitoring devices 30. The monitoring terminal 12 and the mobile monitoring device 30 are capable of being in wireless communication, while the spot measurement device 40 and the mobile monitoring device 30 are also capable of being in wireless communication.

The spot measurement device 40 is configured to detect at least one physiological parameter of a patient according to a user operation, so as to obtain physiological parameter data, and wirelessly transmit the measured data to the mobile monitoring device 30 which is worn by the patient. The spot measurement device 40 enables the mobile monitoring device 30 to obtain the above physiological parameter data in a first wireless transmission manner. The spot measurement device 40 is not a device dedicated to a certain patient, but a physiological parameter measurement device suitable for multiple patients, and can measure basic physiological parameters for all patients in a department. The physiological parameter data, which is measured by the spot measurement device 40, may include at least one of parameter data for body temperature, parameter data for blood pressure, and parameter data for blood glucose. That is, the spot measurement device 40 may be a thermometer, a blood pressure meter, a blood glucose meter, etc., and the nurse measures the body temperature, the blood pressure, and the blood glucose of the patient through the spot measurement device 40, and transmits these data to the mobile monitoring device 30 worn by the patient. As the name suggests, a measurement of the spot measurement device 40 is counted in times, and it may implement several times of measurements one day. The first wireless transmission manner between the spot measurement device 40 and the mobile monitoring device 30 can adopt an existing technology. In an embodiment of this disclosure, the first wireless transmission manner takes short-range communication as an example for explanation, such as Near-field communication (NFC for short). After the spot measurement device 40 measures the physiological parameter data of the patient, the spot measurement device 40 can transmit the physiological parameter data to the mobile monitoring device 30 worn by the patient when it is moved close to the mobile monitoring device 30, which is very convenient and never transmits the data incorrectly. In other implementation methods, the short-range communication is not limited to Near-field communication (NFC for short) technology, but can also include passive/active radio frequency identification (RFID for short) technology, optical, infrared (IR for short) technology, ultra-wideband (UWB for short) technology, magnetic pairing technology, or point-to-point wireless technology, including but not limited to Wi-Fi, Bluetooth, Zigbee or other communication protocols. In an embodiment of this disclosure, the first wireless communication unit is, for example, a Near-field communication (NFC) module, a barcode module, a QR code module, a passive/active radio frequency identification (RFID for short) module, an optical module, an infrared (IR for short) module, an ultra-wideband (UWB for short) module, a magnetic pairing module, or a point-to-point wireless module. The above-mentioned Near-field communication unit may include a reader module and/or a card simulator module.

The spot measurement device 40 enables the mobile monitoring device 30 to obtain the above physiological parameter data in the first wireless transmission manner. For example, the wireless communication unit of one of the spot measurement device 40 and the mobile monitoring device 30 may be configured to continuously transmit out a broadcast signal (becoming a slave device) at a certain time interval (such as a preset time interval). The broadcast signal contains connection information, which can be used by an external device to pair with the slave device to establish communicative connection. For example, the connection information may include: a device identification code (i.e., device ID) or a serial number (SN for short) of any one of the spot measurement device 40 and the mobile monitoring device 30; or may include: one of a communication channel identification code, an IP address, an MAC address, a Service Set Identifier (SSID), WEP (Wired Equivalent Privacy) protocol, WPA (Wi-Fi Protected Access) protocol, WPA2 protocol, WPA3 protocol and EAP (Extensible Authentication Protocol), which are determined by any one of the spot measurement device 40 and the mobile monitoring device 30; or may include a random pairing code which is generated by any one of the spot measurement device 40 and the mobile monitoring device 30. A processor of the other one of the spot measurement device 40 and the mobile monitoring device 30 receives the broadcast signal which is transmitted by the wireless communication unit of the slave device through its wireless communication unit, and then establishes a wireless communicative connection with the slave device.

For example, in one embodiment, the spot measurement device 40 may be a wireless sensor, which is developed by a third-party manufacturer, such as a Bluetooth body temperature patch, a Bluetooth blood glucose patch, a blood pressure monitor, etc. In order to facilitate the integration of their own systems or the integration with systems from other manufacturers, these wireless sensor manufacturers provide a Bluetooth address of the sensor through barcode(s), QR code(s), and text, so that the Bluetooth address can be transmitted to the mobile monitoring device which is worn by the patient through short-range communication. When the spot measurement device and the mobile monitoring device are paired through Near-field communication, the mobile monitoring device, as the Bluetooth master device, requires knowing the Bluetooth address of the spot measurement device, which is to be paired as the slave device, so as to complete the pairing. After the mobile monitoring device contacts the spot measurement device through NFC, it recognizes that it needs to enter into a pairing scenario, and then scans out the closest Bluetooth device to be paired according to a signal strength of the received Bluetooth broadcast signal, that is, the contacted spot measurement device, and then uses the address information inside the Bluetooth broadcast signal to complete the pairing connection.

As the third-party manufacturers of the wireless sensor usually do not provide NFC functionality. Therefore, in order to integrate the spot measurement device developed by third-party manufacturers into the monitoring system, it is necessary to attach a self-prepared NFC tag to a surface of the spot measurement device. When a pairing is required, the spot measurement device which is attached with the NFC tag is firstly contacted with the mobile monitoring device which is worn by the patient and to be paired. At this time, the mobile monitoring device senses the NFC contact and immediately enters into an NFC pairing mode. In the NFC pairing mode, the master device records the signal strength of the Bluetooth broadcast signal of the spot measurement device received within a period of time, and selects a third-party device with the best signal strength (i.e., the spot measurement device) as the pairing object, and uses the address information of the pairing object to actively establish communication pairing with this pairing object.

The monitoring terminal 12 is configured to monitor one patient or multiple patients, and may be, for example, a bedside monitor, a central station (central monitoring workstation), etc.

The mobile monitoring device 30 is worn on a body of the patient, so as to monitor the patient for obtaining monitoring data. A working mode of mobile monitoring device includes a continuous monitoring mode and an intermittent monitoring mode. When the mobile monitoring device 30 is worn on the patient for work, it is also bound with identification information of the patient, which information is configured to uniquely identify the patient and ensure a uniqueness of an ownership of the monitoring data. The identification information may be, for example, patient ID, patient support number, etc.

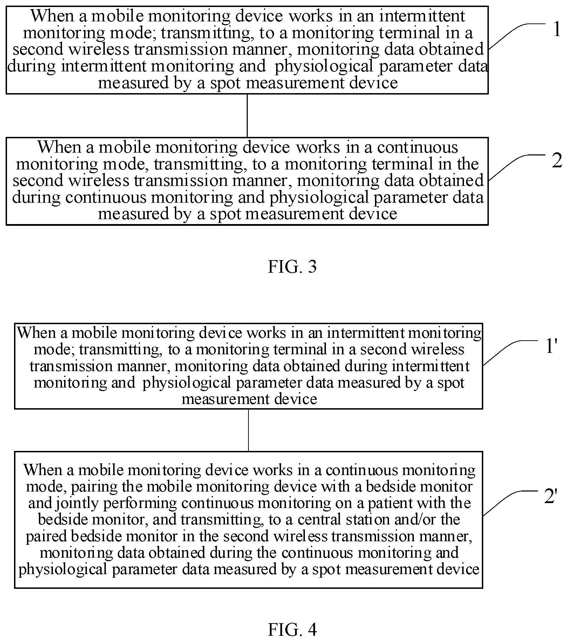

A monitoring method as shown in FIG. 3 of the monitoring system shown in FIG. 1, includes following steps.

In Step 1, the mobile monitoring device 30 works in the intermittent monitoring mode, and performs the intermittent monitoring on the patient (such as intermittently measuring one or more physiological parameters), so as to obtain the monitoring data; and receives, in the first wireless transmission manner, the physiological parameter data of the patient, which data is measured by the spot measurement device 40; and transmits, to the monitoring terminal 12 in the second wireless transmission manner, the monitoring data which is obtained during the intermittent monitoring and the physiological parameter data which is measured by the spot measurement device 40. The medical staff (user) can set the working mode of the mobile monitoring device to the intermittent monitoring mode, or the monitoring terminal 12 can control the mobile monitoring device to work in the intermittent monitoring mode. The second wireless transmission manner can be any existing wireless communication method without limitation. For example, the second wireless transmission manner can be a transmission manner using one or more of following wireless technologies: Bluetooth, Wi-Fi, WMTS (wireless medical telemetry services). The second wireless communication unit can use various types of existing wireless communication modules without limitation. For example, the second wireless communication unit includes at least one of: a Bluetooth module, a WMTS communication module, and a WIFI communication module. The second wireless transmission manner may be different from the first wireless transmission manner. The intermittent monitoring mode is suitable for patients who are getting out of support for recovery after surgery, mild patients under emergency observation, etc. That is, the intermittent monitoring mode is conducive to activities of recovering patients without restricting activities of mild patients under emergency observation. Since the cost of the mobile monitoring device 30 is relatively low and can be used in large quantities, so that patients with mild symptoms can be well monitored, and even if their symptoms are aggravated, such aggravation can be discovered in time and will not be missed. If the symptoms of the patient are milder, the mobile monitoring device 30 may not perform monitoring; but only receive, in the first wireless transmission manner, the physiological parameter data of the patient measured by the sport measurement device 40, and transmit, in the second wireless transmission manner, the physiological parameter data measured by the sport measurement device 40 The transmission manner is transmitted to the monitoring terminal 12, which saves work of entering into the physiological parameter data by the nurse and has a high degree of automation.

In step 2, the mobile monitoring device 30 works in the continuous monitoring mode, and performs the continuous monitoring on the patient (such as continuously measuring one or more physiological parameters), so as to obtain the monitoring data; and receives, in the first wireless transmission manner, the physiological parameter data of the patient, which data is measured by the spot measurement device 40; and transmits, to the monitoring terminal 12 in the second wireless transmission manner, the monitoring data which is obtained during the continuous monitoring and the physiological parameter data which is measured by the spot measurement device 40. Similarly, the medical staff (user) can set the working mode of the mobile monitoring device to the continuous monitoring mode, or the monitoring terminal 12 can control the mobile monitoring device to work in the continuous monitoring mode. If the mild symptoms of the patient are aggravated, the continuous monitoring mode can be used to avoid missing abnormal physiological parameters.

If the monitoring terminal 12 is a bedside monitor, the mobile monitoring device 30 is also paired with the bedside monitor to jointly perform the continuous monitoring on the patient with the bedside monitor; and transmits, to the bedside monitor in the second wireless transmission manner, the monitoring data which is obtained during the continuous monitoring and the physiological parameter data which is measured by the spot measurement device 40. If the symptoms of the patient are more serious, the two devices can be used for continuous monitoring, so as to measure the physiological parameter of the patient in an all-round way, so that medical staff can keep track of the patient state at any time.

In order to better monitor the patient and satisfy different monitoring requirements, as shown in FIG. 2, the monitoring system provided by this disclosure can include both a bedside monitor 20 and a central station 10. The bedside monitor 20 and the central station 10 can be in wired or wireless communication with each.

The bedside monitor 20 is configured to monitor the patient and pair with the mobile monitoring device 30.

A monitoring method as shown in FIG. 4 of the monitoring system shown in FIG. 2, includes following steps.

In Step 1', the mobile monitoring device 30 works in the intermittent monitoring mode, and performs the intermittent monitoring on the patient, so as to obtain the monitoring data; and receives, in the first wireless transmission manner, the physiological parameter data of the patient, which data is measured by the spot measurement device 40; and transmits, to the central station 10 in the second wireless transmission manner, the monitoring data which is obtained during the intermittent monitoring and the physiological parameter data which is measured by the spot measurement device 40. For patients in the postoperative recovery period, emergency patients with mild symptoms, etc., only the mobile monitoring device 30 is configured to monitor them, which does not restrict the free activity of the patients and is conducive to their activity and recovery. Intermittent monitoring is adopted to save power, while satisfying the monitoring requirements of such patients.

The spot measurement device 40 usually does not have the ability to automatically measure, such that the nurse completes the measurement of the physiological parameter of the patient. Therefore, when to measure, and a measurement frequency, etc., are usually uncontrollable. Moreover, only the nurse and the patient himself know the physiological parameter data recorded by the spot measurement device 40 belongs to which patient, while the spot measurement device 40 itself cannot bind the physiological parameter data to the patient. Therefore, after receiving the physiological parameter data measured by the spot measurement device, the mobile monitoring device 30 can associate the physiological parameter data measured by the spot measurement device with the identification information of the patient who wears the mobile monitoring device, that is, to bind the identification information of the patient with the physiological parameter data of the patient, so as to determine a patient ownership of the physiological parameter data, and then transmit the identification information and the physiological parameter data to the central station 10 in the second wireless transmission manner, so that the central station 10 knows which patient the physiological parameter data measured by the spot measurement device 40 belongs to. The mobile monitoring device 30 can wirelessly transmit the physiological parameter data measured by the spot measuring device 40 to the central station 10 after receiving it, or can wirelessly transmit the physiological parameter data measured by the spot measuring device 40 to the central station 10 at intervals, for example, jointly transmitting to the central station 10, identification information, physiological parameter data and self-measured monitoring data of the patient, in a next data transmission cycle.

The monitoring data, which is obtained by the mobile monitoring device 30 through performing the intermittent monitoring on the patient, includes at least one of a physiological parameter value, a physiological parameter curve and an alarm event. Specifically, the mobile monitoring device 30 intermittently measures one or more physiological parameters of the patient, so as to obtain one or more physiological parameter data. For example, the mobile monitoring device 30 intermittently measures at least one of: a blood oxygen parameter, a blood pressure parameter, a pulse rate parameter, a body temperature parameter, an ECG parameter, a respiratory parameter, a sleep parameter, and an exercise parameter, of the patient, so as to obtain at least one of parameter data for the blood oxygen, parameter data for blood pressure, parameter data for pulse rate, parameter data for body temperature, and ECG parameter data, parameter data for respiratory parameter data, sleep parameter data, and exercise parameter data. The above-mentioned physiological parameter data measured by the mobile monitoring device 30 itself includes physiological parameter values and their corresponding measurement times. The mobile monitoring device 30 can only transmit these data to the central station 10, or can further process these data, such as processing physiological parameters at different measurement times to generate a physiological parameter curve. For another example, whether the physiological parameter data (such as a physiological parameter value at each time) exceeds a corresponding threshold, is determined, and if yes, corresponding alarm information is generated. The alarm information can be text, image, or sound, etc. The mobile monitoring device 30 can output the monitoring data to its own display screen for display, and can also output the alarm information to its own speaker for sounding.

The central station 10 receives the physiological parameter data, which is measured by the spot measurement device 40 and transmitted by the mobile monitoring device 30, and displays the physiological parameter data according to the patient. That is, the physiological parameter values of each patient, which values are measured by the spot measurement device can be seen on a display screen of the central station 10. When displaying the physiological parameter data measured by the spot measurement device 40 according to the patient, the display screen of the central station 10 also displays an identifier which is associated with the spot measurement device 40 or also displays a measurement time of the physiological parameter data. The identifier associated with the spot measurement device 40 is configured to identify the spot measurement device 40 , which may be a type, name, device ID of the spot measurement device 40, or a name, support number of the patient, etc. The measurement time of physiological parameter data is the time when the spot measurement device 40 measures the patient and obtains the physiological parameter data. Through the identification or measurement time of the spot measurement device 40, the user can not only see the physiological parameter data from the central station 10, but also can identify the measurement source of the physiological parameter data, that is, the data is measured by the spot measurement device 40 or the mobile monitoring device 30.

The central station 10 receives the monitoring data, which is transmitted by the mobile monitoring device 30. If the monitoring data is the physiological parameter value, physiological parameter curve, alarm event, etc., that has been processed by the mobile monitoring device 30, the central station 10 presents the monitoring data, through directly displaying on its own display screen, or sounding through its own loudspeaker, according to the patient. If the monitoring data is raw data, which is not processed, the central station 10 processes the monitoring data, so as to obtain at least one of the physiological parameter value, physiological parameter curve, alarm event, and then displays the processed data through its own display screen, according to the patient, or feeds back the processed data to the mobile monitoring device 30 for displaying through the display screen of the mobile monitoring device 30 or for sounding through the speaker of the mobile monitoring device 30.

The central station 10 further determines whether the physiological parameter data measured by the spot measurement device 40 exceeds the corresponding threshold, and if yes, outputs corresponding alarm information. The central station 10 further determines whether one or more physiological parameters in the monitoring data of the mobile monitoring device 30 exceed the corresponding threshold, and if yes, outputs corresponding alarm information. When the central station 10 outputs the alarm information, it can display the alarm information on its own display screen or sound out the alarm information through a speaker, according to the patient, and can also feed the alarm information back to the mobile monitoring device 30 of the patient, and the mobile monitoring device 30 of the patient displays or sounds out the alarm information.

In step 2', the mobile monitoring device 30 works in the continuous monitoring mode and pairs with the bedside monitor 20, so as to jointly perform continuous monitoring on the patient with the bedside monitor 20 for obtaining monitoring data; and transmits, to the central station 10 and/or the paired bedside monitor 20 in a second wireless transmission manner, the monitoring data which is obtained during the continuous monitoring and the physiological parameter data which is measured by the spot measurement device. For example, if a patient in the postoperative recovery period encounters an emergency, a bedside monitor 20 can be added to perform a continuously monitoring on the patient.

Adopting the continuous monitoring mode indicates that a close attention needs to be paid to the patient, therefore, in the continuous monitoring mode, the mobile monitoring device 30 can transmit, in real time in the second wireless transmission manner, the physiological parameter data measured by the spot measurement device to the central station 10 and/or the paired bedside monitor 20.

The monitoring data obtained by the mobile monitoring device 30 through performing the continuous monitoring on the patient may also include at least one of a physiological parameter value, a physiological parameter curve and an alarm event. Specifically, the mobile monitoring device 30 continuously measures one or more physiological parameters of the patient in real time, so as to obtain one or more physiological parameter data.

The continuous monitoring mode may, for example, mean that the mobile monitoring device 30 transmits data (monitoring data obtained by the mobile monitoring device 30 and/or physiological parameter data measured by the spot measurement device) to the central station 10 and/or the paired bedside monitor 20, in multiple continuous transmission cycles. In contrast, the intermittent monitoring mode may, for example, mean that the mobile monitoring device 30 only transmits data (monitoring data obtained by the mobile monitoring device 30 and/or physiological data measured by the spot measurement device) to the central station 10 and/or the paired bedside monitor 20, in a portion of transmission cycle(s) of the multiple continuous transmission cycles. As the name suggests, the continuous monitoring mode means that the mobile monitoring device 30 monitors the patient continuously and uninterruptedly, and transmits data to the central station 10 and/or the paired bedside monitor 20. For example, a transmission cycle (such as 1 second, etc., in which the transmission cycle may be a time period required to transmit data once) is set, then in each transmission cycle, the mobile monitoring device 30 in the continuous monitoring mode transmits data.

The monitoring mode, which is opposite to the continuous monitoring mode, is a discontinuous monitoring mode which includes an intermittent monitoring mode, a spot monitoring mode, etc. The intermittent monitoring mode may, for example, mean that the mobile monitoring device 30 transmits the data discontinuously and intermittently after obtaining the monitoring data of the patient. That is, for the previously set transmission cycle, data is not transmitted in every transmission cycle, but is transmitted in a portion of transmission cycle(s), while not transmitted in other transmission cycle(s).

It should be noted that the continuous monitoring mode and intermittent monitoring mode referred to in this disclosure are only relative concepts. The core is that the frequency of data transmission to remote devices (such as target monitoring devices) using the continuous monitoring mode is greater than that using the intermittent monitoring mode. Therefore, the former consumes higher power, so it is usually suitable for more critical patients. When the symptom of the patient improves, the intermittent monitoring mode, which has relatively lower data transmission frequency and power consumption, can be employed.

The mobile monitoring device 30 may also have a spot monitoring mode. Compared with the first two modes, the spot monitoring mode is a data transmission mode with lower power consumption. When the mobile monitoring device 30 works in the spot measurement monitoring mode, it only obtains and transmits data (monitoring data obtained by the mobile monitoring device 30 and/or physiological parameter data measured by the spot measurement device) to the central station 10 and/or paired bedside monitor 20 at a preset designated time, which usually only transmits once or several times (for example, one transmission fails) at the designated time.

In different working modes, the types of the physiological parameters measured by the mobile monitoring device 30 may be the same, but the monitoring frequencies are different. For example, the mobile monitoring device 30 measures the physiological parameters more frequently in the continuous monitoring mode than in the intermittent monitoring mode. Of course, the mobile monitoring device 30 can also transmit monitoring data to the central station 10 and/or paired bedside monitor with a higher frequency in the continuous monitoring mode than that in the intermittent monitoring mode. Both modes can make the monitoring frequencies of the two working modes different. The above-mentioned physiological parameter data measured by the mobile monitoring device 30 itself includes physiological parameter values and their corresponding measurement times. The mobile monitoring device 30 can only transmit these data to the central station 10 and/or the paired bedside monitor 20, or it can further process these data, such as processing physiological parameter values at different measurement times into physiological parameter curve(s), or determining whether the physiological parameter data (such as the physiological parameter value at each time) exceeds the corresponding threshold, and if yes, generating corresponding alarm information. The alarm information can be text, image, or sound, etc. The mobile monitoring device 30 can output monitoring data to its own display screen for display, and can also output alarm information to its own speaker for sounding.

The central station 10 receives the physiological parameter data, which is measured by the spot measurement device and transmitted by the mobile monitoring device 30, and displays it according to the patient. That is, the physiological parameter values measured by the spot measurement device of each patient can be seen on the display screen of the central station 10. The bedside monitor 20 receives the physiological parameter data, which is measured by the spot measurement device and transmitted by the mobile monitoring device 30, and displays the data according to the patient. That is, the physiological parameter values, which are measured by the paired spot measurement device of the patient, can be displayed on the display screen of the bedside monitor 20 .

The central station 10 and/or the paired bedside monitor 20 receive(s) the monitoring data transmitted by the mobile monitoring device 30. If the monitoring data is the physiological parameter value, physiological parameter curve, alarm event, etc., that has been processed by the mobile monitoring device 30, the central station 10 and/or the paired bedside monitor 20 present(s) the monitoring data, through directly displaying on their/its own display screen, or sounding through their/its own loudspeaker, according to the patient. If the monitoring data is raw data, which is not processed, the central station 10 and/or the paired bedside monitor 20 process(es) the monitoring data, so as to obtain at least one of the physiological parameter value, physiological parameter curve, alarm event, and then display(s) the processed data through their/its own display screen, according to the patient, or feed(s) the processed data back to the mobile monitoring device 30 for displaying through the display screen of the mobile monitoring device 30 or for sounding through the speaker of the mobile monitoring device 30. The central station 10 can also transmit at least one of the physiological parameter value, the physiological parameter curve, the alarm event, to the bedside monitor 20 of the patient for displaying through the display screen of the bedside monitor 20 or for sounding through the speaker of the bedside monitor 20. The bedside monitor 20 can also transmit at least one of the physiological parameter value, the physiological parameter curve, the alarm event, to the central station 10 of the patient for displaying through the display screen of the central station 10 or for sounding through the speaker of the central station 10.

The central station 10 and/or the paired bedside monitor 20 further determine(s) whether the physiological parameter data, which is measured by the spot measurement device 40 exceeds the corresponding threshold, and if yes, output(s) corresponding alarm information. The central station 10 and/or the paired bedside monitor 20 further determine(s) whether one or more physiological parameters in the monitoring data of the mobile monitoring device 30 exceed the corresponding threshold, and if yes, output(s) corresponding alarm information. When the central station 10 outputs the alarm information, it can display the alarm information through its own display screen according to the patient, or sound out the alarm information through a speaker according to the patient; and can also feed the alarm information back to the mobile monitoring device 30 of the patient and/or the bedside monitor 20 of the patient, and enable the mobile monitoring device 30 and/or the bedside monitor 20 to display or sound out the alarm information. When the bedside monitor 20 outputs the alarm information, it can display the alarm information through its own display screen according to the patient, or sound out the alarm information through a speaker according to the patient; and can also feed the alarm information back to the mobile monitoring device 30 of the patient and/or the central station 10 of the patient, and enable the mobile monitoring device 30 and/or the central station 10 to display or sound out the alarm information.

In the monitoring system, a number of mobile monitoring devices 30 is greater than a number of bedside monitor(s) 20. The mobile monitoring device 30 and the bedside monitor 20 do not need to be paired (bound) one by one and can be paired when necessary. When working, some mobile monitoring devices 30 are paired with one bedside monitor 20 and jointly work with said bedside monitor 20, while some mobile monitoring devices 30 are not paired with any bedside monitor 20.

After a first bedside monitor and a first mobile monitoring device pair with each other and jointly work, the first bedside monitor is disconnected from the first mobile monitoring device, so as to pair with a second mobile monitoring device, which is different from the first mobile monitoring device, and jointly work with the second mobile monitoring device.

Of course, the bedside monitor 20 may not need to pair with the mobile monitoring device 30 to work. For example, just after surgery, the patient cannot get out of support and does not need to wear the mobile monitoring device 30, the bedside monitor 20 can be used alone to perform a continuously monitoring on the patient. The bedside monitor 20 measures at least one of a blood oxygen parameter, a blood pressure parameter, a pulse rate parameter, a body temperature parameter, an ECG parameter, a respiratory parameter, and a sleep parameter, so as to obtain the monitoring data.

The mobile monitoring device 30 can also work in the continuous monitoring mode but does not pair with the bedside monitor 20. For example, if the patient is not at the bedside and cannot be paired with the bedside monitor 20, or there is no need to pair with the bedside monitor 20, then the mobile monitoring device 30 independently monitors the patient continuously, transmits the monitoring data obtained during the continuous monitoring and the physiological parameter data measured by the spot measurement device to the central station in the second wireless transmission manner. The specific process and transmission of the monitoring data are the same as above and is not described in detail.

In the physiological parameters, which are measured by the mobile monitoring device 30 through monitoring the patient, and the physiological parameters, which are measured by the bedside monitor 20 through monitoring the patient, at least some of the physiological parameters are the same. In this way, after the patient releases the bedside monitor 20, the relevant physiological parameters of the patient can be monitored by the mobile monitoring device 30, so as to ensure a safety of the patient.

To sum up, the monitoring system of this disclosure forms corresponding combinations of the spot measurement device, mobile monitoring device, bedside monitors, and central station, according to different clinical applications. There are even five levels of patient monitoring: spot measurement device only, spot measurement device + mobile monitoring device in an intermittent monitoring mode, spot measurement device + mobile monitoring device in continuous monitoring, bedside monitor monitoring, spot measurement device + mobile monitoring device in continuous monitoring + bedside monitor monitoring; which are capable of flexibly adapting to the monitoring requirements of patients with different severity levels. For example, when a patient is in a critical period, the mobile monitoring device can be quickly paired and connected with the bedside monitor to form a jointly continuous monitoring mode, so as to achieve a high-level continuous monitoring of the patient. When the patient gradually recovers, the bedside monitor can be removed, so as to form a mobile monitoring mode of the mobile monitoring device, which mode is separate and intermittent. For a patient at the end of recovery, the spot measurement device can be used to measure basic parameters of the patient on a regular frequency, so as to record basic vital signs. Data from the spot measurement device can be quickly transmitted wirelessly to the mobile monitoring device, bedside monitor, and central station. Data from the spot measurement device, the mobile monitoring device and the bedside monitor can also be transmitted directly to the central station wirelessly to facilitate centralized viewing of clinical staff. The monitoring system thus formed conforms to actual work scenarios, satisfies most patient measurement requirements in sub-critical care departments, and improves work efficiency.

The above-mentioned monitoring system is applicable in a variety of application scenarios. Here are a few examples to illustrate.

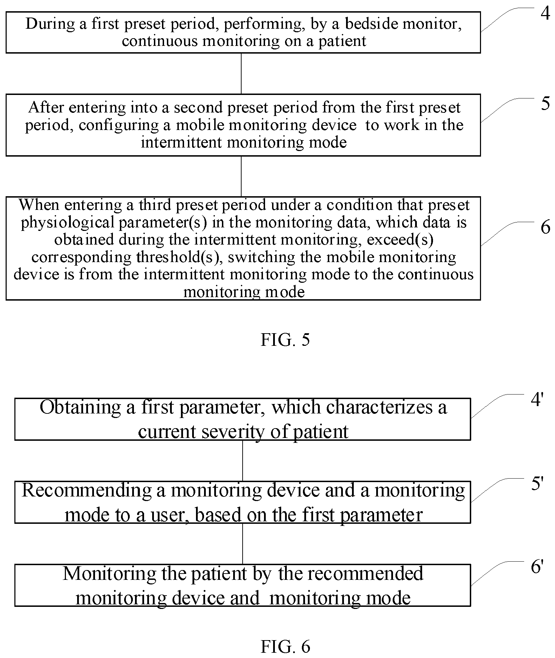

For example, it can be used in postoperative patient monitoring scenarios. As shown in FIG. 5, the monitoring process includes the following steps.

In step 4, during a first preset period, the bedside monitor 20 continuously monitors the patient. The first preset period can be a bedridden risk period after surgery. For patients who have just returned to the general ward and are in the bedridden risk period, the continuous monitoring mode can be adopted. Whether the current patient is in the first preset period, can be determined based on a user operation. For example, the user binds the bedside monitor 20 to the patient, so as to enable the bedside monitor 20 to perform a bedside monitoring on the patient. At this time, it can be determined as the current patient is in the first preset period by default. Of course, The user can further input relevant content or instruction to the bedside monitor 20 or the central station 10, so that the bedside monitor 20 and the central station 10 determine that the current patient is in the first preset period. Usually, the patient is bedridden during this period and the illness condition of the patient is unstable. The bedside monitor 20 can be used alone to perform a continuously monitoring on the patient. The bedside monitor 20 can monitor at least one of the blood oxygen parameter, the blood pressure parameter, the pulse rate parameter, the body temperature parameter, the ECG parameter, the respiratory parameter, and the sleep parameters of the patient, so as to obtain monitoring data. Of course, during the first preset period, the mobile monitoring device 30 can also be worn on the patient and paired with the bedside monitor 20, so as to jointly perform the continuous monitoring on the patient. After the mobile monitoring device 30 and the bedside monitor 20 are paired, the mobile monitoring device 30 and/or the bedside monitor 20 transmit(s) pairing information to the central station 10. After the mobile monitoring device 30 and the bedside monitor 20 are paired, the working mode can be automatically set as the continuous monitoring mode. Optionally, the paired bedside monitor 20 can also control the mobile monitoring device 30 to set the working mode of the mobile monitoring device 30 to the continuous monitoring mode. Optionally, the central station 10 can also control the mobile monitoring device 30 to set the working mode of the mobile monitoring device 30 to the continuous monitoring mode. Optionally, the medical staff can also set the working mode of the mobile monitoring device 30 to the continuous monitoring mode. The mobile monitoring device 30 wirelessly transmits the monitoring data obtained through the continuous monitoring to the central station 10 and/or the paired bedside monitor 20.

In step 5, after entering into the second preset period from the first preset period, the mobile monitoring device 30 works in the intermittent monitoring mode, performs the intermittent monitoring on the patient, and wirelessly transmits the monitoring data obtained during the intermittent monitoring to the central station 10. The second preset period may be an out-of-support recovery period. Specifically, under trigger(s) of a first preset condition and/or an instruction to switch the working mode, it is determined as entering into the second preset period from the first preset period, the mobile monitoring device 30 is switched from the continuous monitoring mode to the intermittent monitoring mode. Under the trigger(s) of a first preset condition and/or an instruction to switch the working mode, the mobile monitoring device 30 can be automatically switched from the continuous monitoring mode to the intermittent monitoring mode, or can be switched from the continuous monitoring mode to the intermittent monitoring mode under a control of the central station 10 or the bedside monitor 20. Wherein the first preset condition can be set as needed. For example, the first preset condition can be that a duration, in which the mobile monitoring device 30 works in the continuous monitoring mode, exceeds a preset time length. Take the preset time length as 8 hours for example, that is, enter into the second preset period automatically, when the first preset period lasts for more than 8 hours. For another example, the first preset condition can be reaching a preset time. For example, the preset time is 8 a.m. of a next day. After the surgery, automatically enter into the second preset period at eight o'clock the next morning. For another example, the first preset condition may be that entering into the second preset period by default, when the mobile monitoring device 30 is disconnected from the paired bedside monitor 20. In this embodiment, in order to improve safety, the first preset condition also includes that an EWS score of the patient is lower than a preset score. The EWS score is lower than the preset score, indicating that the symptom of the patient is stable. On this basis, the continuous monitoring mode is switched to the intermittent monitoring, only when the duration exceeds the preset time length, the preset time is reached, or the paired bedside monitor 20 is disconnected.

The bedside monitor 20 and/or the mobile monitoring device 30 are further configured to unpair the bedside monitor 20 and the mobile monitoring device 30, under trigger(s) of a first preset condition and/or an instruction to switch the working mode. Then, the mobile monitoring device 30 performs the intermittent monitoring on the patient alone. Of course, the central station 10 can also control the bedside monitor 20 and/or the mobile monitoring device 30 to unpair, under trigger(s) of a first preset condition and/or an instruction to switch the working mode.

In step 6, in the monitoring data obtained in the intermittent monitoring mode, if a preset physiological parameter exceeds a corresponding threshold, enter into a third preset period. At this time, it means that the patient is abnormal and may have a recrudescent condition, and requires to be monitored more. The mobile monitoring device 30 is switched from the intermittent monitoring mode to the continuous monitoring mode. Whether the physiological parameter exceeds the corresponding threshold can be determined by the mobile monitoring device 30 or the central station 10, which has been explained in detail in the above embodiment. The monitoring data of the mobile monitoring device 30 includes the preset physiological parameter. The preset physiological parameter is more important, and its abnormity reflects that the symptom of the patient has become serious. Medical staff can preset the specific type of the preset physiological parameter. In this embodiment, the preset physiological parameter is an electrocardiographic parameter. The mobile monitoring device 30 determines whether the ECG parameter exceeds the corresponding threshold. If yes, it determines that the patient has malignant arrhythmia, generates a preset message, and transmits it to the central station, and outputs corresponding alarm information.

There are two situations in the third preset period. One is that the patient is outside the ward and the mobile monitoring device 30 cannot be paired with the bedside monitor 20. Then the mobile monitoring device 30 works in the continuous monitoring mode and monitors the patient alone. The other is that the patient is in the ward, or after the patient returns to the ward, the mobile monitoring device 30 pairs with the bedside monitor 20 to jointly perform the continuous monitoring on the patient. When the mobile monitoring device 30 is switched from the intermittent monitoring mode to the continuous monitoring mode, it can be automatically switched after the preset physiological parameter exceeds the corresponding threshold; or can be automatically switched after being paired with the bedside monitor 20; or can be switched by the central station 10 or the paired bedside monitor 20 after the preset physiological parameter exceeds the corresponding threshold; or can be switched by the central station 10 or the paired bedside monitor 20 after being paired with the bedside monitor 20. Of course, the mobile monitoring device 30 can also be switched by medical staff.

EWS score can also be introduced to switch working modes or assist in working mode switching.

Specifically, when the mobile monitoring device 30 does not pair with the bedside monitor 20 and works alone in the intermittent monitoring mode, the central station is configured to obtain a current EWS score of the patient (early warning score); and control the mobile monitoring device of the patient to be switched from the intermittent monitoring mode to the continuous monitoring mode, or to output information, which indicates the user to pair the mobile monitoring device 30 with the bedside monitor 20 for working jointly and to use the continuous monitoring mode; when the EWS score is higher than the preset score.

When the mobile monitoring device 30 does not pair with the bedside monitor 20 and works alone in the continuous monitoring mode, the central station 10 is further configured to obtain the current EWS score of the patient, and control the mobile monitoring device to switch from the continuous monitoring mode to the intermittent monitoring mode, when the EWS score is lower than the preset score.

When the mobile monitoring device 30 pairs with the bedside monitor 20 and jointly work in the continuous monitoring mode, the central station 10 is further configured to obtain the current EWS score of the patient; and control the mobile monitoring device to switch from the continuous monitoring mode to the intermittent monitoring mode, and/or to output information which indicates the user to disconnect the mobile monitoring device and the paired bedside monitor, when the EWS score is lower than the preset score.

The preset score can be set as needed, for example, it can be 3 points. Recommending appropriate patient monitoring device and monitoring mode based on EWS scores improves the work efficiency of medical staff.

It can be seen that in this scenario, there are monitoring methods and devices suitable for patients at different stages after surgery, which can not only detect potential risks of the patient in time, but also do not affect the activities of the patient out of bed.

The above-mentioned monitoring system can also be used in emergency monitoring scenarios. As shown in FIG. 6, the monitoring process includes the following steps.

In step 4', a first parameter, which characterizes a current severity of the patient, is obtained, wherein the first parameter can be determined by humans or automatically, which are introduced one by one below.

The central station 10 includes a human-machine interaction apparatus, which is configured for human-machine interaction, such as outputting visual information and receiving user input. The human-machine interaction apparatus can use a keyboard, operation buttons, mouse, trackball, touch pad, etc., as well as a touch screen integrated with the display screen, to receive the user input; and can also use the display screen to output the visual information. The central station 10 receives the first parameter, which is inputted by the user, through its human-machine interaction apparatus, wherein the first parameter characterizes a current severity of the patient. The first parameter can be embodied in various forms, for example various types of potential critical illness scoring systems can be used, such as Early Warning Score (EWS score), Rapid Acute Physiology Score (RAPS score), Rapid Emergency Medicine Score (REMS score) ), Mainz Emergency Evaluation Score (MEES score, which is a dynamic evaluation of an effect and quality of pre-hospital first aid and emergency resuscitation), and other scores that can reflect the severity of the patient, as if all medical staff can use one standard.

This embodiment uses EWS score as an example for explanation. The EWS score helps medical staff to dynamically monitor changes in illness condition and detect potentially critically ill patients early. After receiving the patient, the medical staff can score the patient according to the EWS score standards and input the EWS score into the central station 10. After receiving the patient, the medical staff can also wear the mobile monitoring device 30 on the patient. The mobile monitoring device 30 monitors the patient according to the monitoring mode (working mode) set by the medical staff or according to the monitoring mode preset by the system, so as to obtain monitoring data. The mobile monitoring device 30 transmits the monitoring data to the central station 10. The central station 10 receives the monitoring data transmitted by the mobile monitoring device 30 and obtains current EWS score of the patient based on the monitoring data. That is, the central station 10 can score the patient according to the preset EWS score standards, based on multiple physiological parameters of the patient measured by the mobile monitoring device 30, so as to obtain the current EWS score of the patient. There is no need for medical staff to make the determination, which reduces the workload of medical staff.

In step 5, a monitoring device and a monitoring mode are recommended to the user, based on the first parameter. For example, when the first parameter is lower than a preset first threshold, the central station 10 outputs first information, which indicates the user to use the mobile monitoring device to perform intermittent monitoring on the patient. The first parameter is lower than or equal to the first threshold, indicating that the symptoms of the patient are mild, and the mobile monitoring device can be used for the intermittent monitoring, and the patient can move freely. When the first parameter is higher than the preset first threshold, the central station 10 outputs information which indicates the user to use a bedside monitor and/or a mobile monitoring device to perform a continuously monitoring on the patient. If the first parameter is higher than the first threshold, it may be necessary to continuously monitor and evaluate the patient. Specifically, the central station 10 can output third information which indicates the user to use the mobile monitoring device 30 to perform a continuously monitoring on the patient, or can also output second information which indicates the user to use the bedside monitor 20 to perform a continuously monitoring on the patient. Subdivided processing can be employed based these. This embodiment takes the subdivided processing as an example. In one embodiment, step 5' may include the following steps.

In step 51', the central station 10 determines whether the first parameter is higher than the preset first threshold. If yes, proceed to step 53', if not, proceed to step 52'.

In step 52', when the first parameter is lower than the preset first threshold, the central station 10 outputs first information which indicates the user to use the mobile monitoring device to perform intermittent monitoring on the patient. Wherein, the first information may be at least one of text, graph, and sound. When the central station 10 outputs the first information, the central station 10 may output the first information to the display screen of its human-machine interaction apparatus for displaying; or may output the first information to its speaker for sounding; or may also output the first information to the mobile monitoring device 30 worn by the patient for displaying through the display screen of the mobile monitoring device 30 or for sounding by the speaker of the mobile monitoring device 30; or may also output the first information to the bedside monitor 20 for displaying through the display screen of the bedside monitor 20 or for sounding by the speaker of the bedside monitor 20; so as to present the first information to the medical staff quickly and easily.

In step 53', the central station 10 determines whether the first parameter is higher than a preset second threshold, if yes, proceed to step 55', if not, proceed to step 54'.

In step 54', when the first parameter is between the first threshold and the second threshold, the central station 10 outputs third information which indicates the user to use the mobile monitoring device 30 to perform continuous monitoring on the patient. The first threshold and the second threshold can be set according to the user requirement, and the second threshold only requires to be higher than the first threshold. For example, in one embodiment, the first parameter is between the first threshold and the second threshold, which indicates that the patient may not need hospitalization, but may subsequently change to need hospitalization, so the patient needs a mobile continuous monitoring. That is to say, the free activity of the patient is not restricted, and physiological parameters of the patient can be grasped at any time, and the monitoring standard is appropriate. Similarly, the third information may be at least one of text, graph, and sound. When the central station 10 outputs the third information, the central station 10 may output the third information to the display screen of its human-machine interaction apparatus for displaying; or may output the third information to its speaker for sounding; or may also output the third information to the mobile monitoring device 30 worn by the patient for displaying through the display screen of the mobile monitoring device 30 or for sounding by the speaker of the mobile monitoring device 30; or may also output the third information to the bedside monitor 20 for displaying through the display screen of the bedside monitor 20 or for sounding by the speaker of the bedside monitor 20.

In step 55', when the first parameter is higher than the second threshold, or when the first parameter is equal to the second threshold, the central station 10 outputs fourth information which indicates the user to use the bedside monitor 20 and the mobile monitoring device 30 to jointly perform the continuous monitoring on the patient. In one embodiment, if the first parameter is higher than or equal to the second threshold, it means that the risk on symptom deterioration of the patient has increased, there is a risk of "potential critical illness", and the risk of being admitted to a specialist ward or even an ICU has increased, so two devices are used to perform the continuous monitoring, which can achieve a good monitoring effect. Similarly, the fourth information may be at least one of text, graph, and sound. When the central station 10 outputs the fourth information, the central station 10 may output the fourth information to the display screen of its human-machine interaction apparatus for displaying; or may output the fourth information to its speaker for sounding; or may also output the fourth information to the mobile monitoring device 30 worn by the patient for displaying through the display screen of the mobile monitoring device 30 or for sounding by the speaker of the mobile monitoring device 30; or may also output the fourth information to the bedside monitor 20 for displaying through the display screen of the bedside monitor 20 or for sounding by the speaker of the bedside monitor 20; so as to present the fourth information to the medical staff quickly and easily.

In addition to the first information, the third information and the fourth information, the second information may also be at least one of text, graph, and sound. When the central station 10 outputs the second information, the central station 10 may output the second information to the display screen of its human-machine interaction apparatus for displaying; or may output the second information to its speaker for sounding; or may also output the second information to the mobile monitoring device 30 worn by the patient for displaying through the display screen of the mobile monitoring device 30 or for sounding by the speaker of the mobile monitoring device 30; or may also output the second information to the bedside monitor 20 for displaying through the display screen of the bedside monitor 20 or for sounding by the speaker of the bedside monitor 20.

Since the first parameter in this embodiment uses the EWS score, the corresponding first threshold is a first score, and the second threshold is a second score. The first score and the second score can be set according to the requirements of the user. For example, in one embodiment, the first score is 3 points, and the second score is 5 points. An EWS score of 3 points indicates that medical staff are reminded to evaluate and adjust the treatment plan. An EWS score of 5 points is the best critical point for identifying a severity of the patient. An EWS score of or above 5 points, requires hospitalization, while an EWS score below 5 points usually does not require hospitalization.

In step 6', the patient is monitored by a recommended monitoring device and a monitoring mode. Specifically, the working modes of the mobile monitoring device 30 include a continuous monitoring mode and an intermittent monitoring mode. The working mode of the mobile monitoring device 30 can be set as the recommended working mode by medical staff, or the central station or bedside monitor can control the mobile monitoring device 30 to switch to the corresponding working mode. The specific process has been elaborated in the above embodiments. No further details are given here.

Subsequently, the first parameter of the patient can be continuously monitored, that is, after step 6', return to step 4'. If the first parameter changes, the monitoring device and monitoring mode is recommended to the user again. Regardless of whether the patient is getting better or worse, there is a suitable monitoring method, and the user can make the adjustment based on the recommended monitoring manner, such that patient can receive the most suitable monitoring at different times.

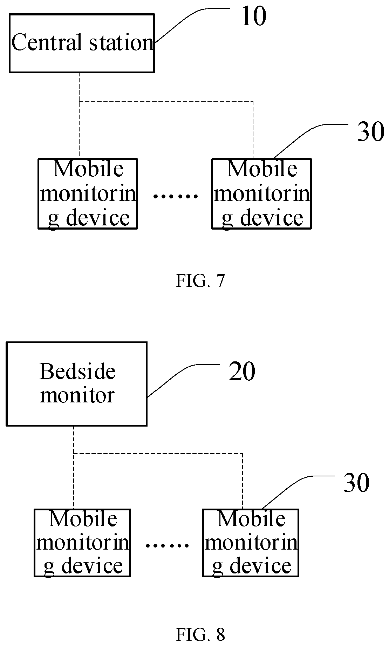

In some embodiments, as shown in FIG. 7, the monitoring system includes the central station 10 as mentioned above and the mobile monitoring device 30 as mentioned above.

The mobile monitoring device 30 is worn on a body of the patient, so as to monitor the patient for obtaining monitoring data.

The working modes of the mobile monitoring device 30 include a continuous monitoring mode and an intermittent monitoring mode. The mobile monitoring device 30 is further configured to perform intermittent monitoring on the patient when working in the intermittent monitoring mode, and receive, in a first wireless transmission manner, physiological parameter data of the patient, which data is measured by a spot measurement device; and transmit, to the central station 10 in the second wireless transmission manner, monitoring data which is obtained during the intermittent monitoring and the physiological parameter data which is measured by the spot measurement device; or perform continuous monitoring on the patient when working in the continuous monitoring mode; and transmit, to the central station 10, monitoring data which is obtained during the intermittent monitoring and the physiological parameter data which is measured by the spot measurement device. The specific monitoring method is shown in FIG. 3, wherein the monitoring terminal in FIG. 3 corresponds to the central station 10. In some embodiments, the monitoring system may also include the spot measurement device 40 as described above. The specific functions of the central station 10, the mobile monitoring device 30 and the spot measurement device 40, as well as, the process in which the three devices cooperate with each other to monitor the patient, have been described in detail in the above embodiments and are not described again here.

In some embodiments, as shown in FIG. 8, the monitoring system includes the bedside monitor 20 as mentioned above and the mobile monitoring device 30 as mentioned above.

The mobile monitoring device 30 is worn on the body of the patient, so as to monitor the patient for obtaining monitoring data. The working modes of the mobile monitoring device 30 include a continuous monitoring mode and an intermittent monitoring mode. The mobile monitoring device 30 is further configured to perform intermittent monitoring on the patient when working in the intermittent monitoring mode, and receive, in a first wireless transmission manner, physiological parameter data of the patient, which data is measured by a spot measurement device; and transmit, to the bedside monitor 20 in a second wireless transmission manner, monitoring data which is obtained during the intermittent monitoring and the physiological parameter data which is measured by the spot measurement device; or pair with the bedside monitor 20 and jointly perform continuous monitoring on the patient with the bedside monitor 20, when working in the continuous monitoring mode; and transmit, to the paired bedside monitor 20 in the second wireless transmission manner, monitoring data which is obtained during the continuous monitoring and physiological parameter data which is measured by a spot measurement device 40. The specific monitoring method is shown in FIG. 3, wherein the monitoring terminal in FIG. 3 corresponds to the bedside monitor 20. In some embodiments, the monitoring system may also include the spot measurement device 40 as described above. The specific functions of the bedside monitor 20, the mobile monitoring device 30 and the spot measurement device 40, as well as, the process in which the three devices cooperate with each other to monitor the patient, have been described in detail in the above embodiments and are not described again here.

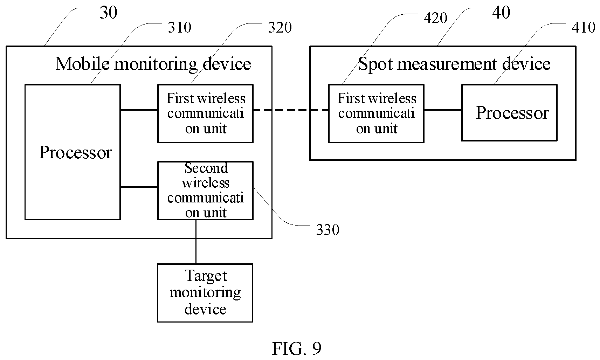

In some embodiments, as shown in FIG. 9, the monitoring system includes the mobile monitoring device 30 as mentioned above and the spot measurement device 40 as mentioned above.

The mobile monitoring device 30 is worn on a body of the patient, so as to monitor the patient and obtain first monitoring data. The mobile monitoring device 30 is in communicative connection with a target monitoring device. For example, the monitoring device paired with the mobile monitoring device 30 is the target monitoring device.

The mobile monitoring device 30 includes a processor 310, a first wireless communication unit 320 and a second wireless communication unit 330.

The processor 310 is configured to receive, in a first wireless transmission manner through the first wireless communication unit 320, the physiological parameter data of the patient, which data is measured by the spot measurement device 40, and to transmit, to the target monitoring device in a second wireless transmission manner through the second wireless communication unit 330, the first monitoring data and the received physiological parameter data of the patient, which data is measured by the spot measurement device 40. Wherein the first wireless communication unit 320 and the second wireless communication unit 330 use different wireless communication protocols; and the first wireless transmission manner is different from the second wireless transmission manner. The target monitoring device may be a central station, a bedside monitor as described above, or another mobile monitoring device, etc.

The processor 310 is further configured to receive, in a first wireless transmission manner through the first wireless communication unit 320, information which is related to the patient, and jointly transmit, to the target monitoring device in a second wireless transmission manner through the second wireless communication unit 330, the information which is related to the patient, the first monitoring data, and the received physiological parameter data of the patient, which data is measured by the spot measurement device 40. Wherein, "jointly transmit to the target monitoring device" can mean that these three types of data (information which is related to the patient, first monitoring data, and physiological parameter data of the patient, which data is measured by the spot measurement device 40) are packaged into one data package and transmitted to the target monitoring device. Of course, these three types of data can also be transmitted to the target monitoring device separately. Moreover, a binding relationship between these data and the patient can be established through "associated information (such as, a same string, a random code, a patient name, a support number, etc., which are transmitted to the target monitoring device; or list information which express a correlation between the three, wherein the list information can also be stored in the target monitoring device in advance without having to be obtained it by "transmission"). That is, let the target monitoring device to know that which patient the received information belongs to.

Similarly, the working modes of the mobile monitoring device 30 include the continuous monitoring mode and the intermittent monitoring mode. The processor 310 is further configured to control the mobile monitoring device 30 to perform intermittent monitoring on the patient; and receive, through the first wireless communication unit 320 in the first wireless transmission manner, the data measured by the spot measurement device 40, when the mobile monitoring device 30 works the intermittent monitoring mode; and to transmit, to the target monitoring device in the second wireless transmission manner, monitoring data, which is obtained during the intermittent monitoring and the physiological parameter data, which is measured by the spot measurement device 40.

Before transmitting data to the target monitoring device, the mobile monitoring device 30 first establishes a communicative connection with the target monitoring device. For example, the processor 310 is further configured to obtain connection information from the target monitoring device in the first wireless transmission manner through the first wireless communication unit 320. The mobile monitoring device 30 may include one or more (two or more) first wireless communication units 320. In this embodiment, the first wireless communication unit 320 uses an NFC module to implement contactless communication with the spot measurement device 40 and the bedside monitor. If the mobile monitoring device 30 includes one first wireless communication unit 320, the same first wireless communication unit 320 is configured to transmit data in the first wireless transmission manner to the spot measurement device 40 and the bedside monitor. If the mobile monitoring device 30 includes multiple first wireless communication units 320, different first wireless communication units 320 are configured to transmit data in the first wireless transmission manner to the spot measurement device 40 and the bedside monitor.

The connection information may include: a device identification code (i.e., device ID) or a serial number (SN for short) of any one of the target monitoring device and the mobile monitoring device 30; or may include: one of a communication channel identification code, an IP address, an MAC address, a Service Set Identifier (SSID), WEP (Wired Equivalent Privacy) protocol, WPA (Wi-Fi Protected Access) protocol, WPA2 protocol, WPA3 protocol and EAP (Extensible Authentication Protocol), which are determined by any one of the target monitoring device and the mobile monitoring device 30; or may include a random pairing code which is generated by the target monitoring device or the mobile monitoring device 30.

Of course, in some embodiments, the processor 310 may also transmit connection information to the target monitoring device through the first wireless communication unit 320. For example, the processor 310 broadcasts the connection information through the first wireless communication unit 320.

Based on the connection information, the processor 310 establishes a communicative connection with the target monitoring device in the second wireless transmission manner through the second wireless communication unit 330; and then based on the communicative connection data interaction with the target monitoring device is performed through the second wireless communication unit. For example, the information which is related to the patient, the first monitoring data, and the physiological parameter data of the patient, which data is measured by the sport measurement device 40 are transmitted to the target monitoring device.

In this embodiment, the connection information is a random pairing code, which is generated by the target monitoring device. The random pairing code may be one code or a pair of codes. Wherein, the target monitoring device generates one code means that the pairing codes between the target monitoring device and the mobile monitoring device 30 are exactly the same, for example, the code is 1111. The target monitoring device generates a pair of codes means that the target monitoring device and the mobile monitoring device 30 have different pairing codes which have a corresponding relationship. For example, the random pairing codes generated by the target monitoring device are 1111 and 2222 (for example, a pairing relationship list is established through an array), wherein the random pairing code 1111 is transmitted to the mobile monitoring device 30, while the random pairing code 2222 is the code paired with 1111. Based on this pair of codes, a communicative connection can be established.

As shown in FIG. 9, a spot measurement device 40 provided by an embodiment includes a processor 410 and a first wireless communication unit 420.

The processor 410 of the spot measurement device 40 is configured to transmit, to the mobile monitoring device 30 in a first wireless transmission manner through the first wireless communication unit 420, the physiological parameter data of the patient, which data is measured by the spot measurement device 40, so as to enable the mobile monitoring device 30 to transmit, to a target monitor device in a second wireless transmission manner through a second wireless communication unit which is arranged at the mobile monitoring device 30, the received physiological parameter data of the patient, which data is measured by the spot measurement device. Specifically, the working modes of the mobile monitoring device include a continuous monitoring mode and an intermittent monitoring mode. When the mobile monitoring device 30 works in the intermittent monitoring mode, the processor 410 of the spot measurement device 40 is configured to transmit, to the mobile monitoring device 30 in the first wireless transmission manner through the first wireless communication unit 420, the physiological parameter data of the patient, which data is measured by the spot measurement device, so as to enable the mobile monitoring device 30 to transmit, to the target monitor device in the second wireless transmission manner through the second wireless communication unit which is arranged at the mobile monitoring device 30, the received physiological parameter data of the patient, which data is measured by the spot measurement device. Wherein, the first wireless communication unit 420 of the spot measurement device 40 is adapted to the first wireless communication unit 320 of the mobile monitoring device 30, and the two communication units communicate in the first wireless transmission manner. For details, refer to the above description. In this embodiment, the first wireless transmission manner is different from the second wireless transmission manner. Correspondingly, the first wireless communication unit of the spot measurement device 40 and the mobile monitoring device 30 and the second wireless communication unit 330 of the mobile monitoring device 30 adopt different wireless communication protocols.

The first wireless communication unit 420 of the spot measurement device 40 can be an NFC module. Information which is related to the patient (such as, support number, device number, random code, etc., as long as a binding relationship can be established with the patient) can be written in the NFC module. The mobile monitoring device 30 can obtain information which is related to the patient when it moves close to the spot measurement device 40, which is similar to a swiping process of a bus card at a bus card reader or a swiping process of a bank card at a POS machine. At the same time, the mobile monitoring device 30 can also obtain the physiological parameter data, which is obtained by the spot measurement device 40 (such as body temperature, blood oxygen and blood pressure, etc.), so that the mobile monitoring device 30 transmits the two data to the target monitoring device, so that the target monitoring device knows which patient the body temperature value comes from. The entire process is quite simple and efficient, with a high degree of automation.

The process of the mobile monitoring device 30 and the above-mentioned spot measurement device 40 cooperating with each other to monitor the patient has been described in detail in the above embodiments, and is not described again here.

Those skilled in the art can understand that all or part of the functions of various methods in the above embodiments can be implemented by hardware or by computer programs. When all or part of the functions in the above embodiments are implemented by a computer program, the program can be stored in a computer-readable storage medium. The storage medium can include a read-only memory, a random-access memory, a magnetic disk, an optical disk, a hard disk, etc., and the above functions can be achieved through execution of this program through a computer. For example, the program is stored in a device memory, and when the program in the memory is executed by the processor, all or part of the above functions can be realized. In addition, when all or part of the functions in the above embodiments are implemented by a computer program, the program can also be stored in a storage medium, such as a server, another computer, a magnetic disk, an optical disk, a flash disk or a mobile hard disk, and can be downloaded or copied to save it into the memory of the local device, or to perform a version update on the system of the local device. When the program in the memory is executed by the processor, all, or part of the functions in the above embodiments can be realized.

The description has been made with reference to various exemplary embodiments herein. However, those skilled in the art recognize that changes and modifications can be made to the exemplary embodiments without departing from the scope herein. For example, various operation steps and components for performing operation steps may be implemented in different ways according to a specific application or considering any number of cost functions associated with the operation of the system (for example, one or more steps may be deleted, modified, or incorporated into other steps).

In addition, as understood by those skilled in the art, the principles herein may be reflected in a computer program product on a computer-readable storage medium that is pre-installed with computer-readable program codes. Any tangible, non-transitory computer-readable storage medium can be used, including magnetic storage devices (hard disks, floppy disks, etc.), optical storage devices (CD-ROM, DVD, Blu Ray disks, etc.), flash memory, and/or the like. These computer program instructions can be loaded onto a general-purpose computer, a dedicated computer, or other programmable data processing device to form a machine, so that these instructions, which are executed on a computer or other programmable data processing apparatus, can generate an apparatus that implements a specified function. These computer program instructions can also be stored in a computer-readable memory that can instruct a computer or other programmable data processing device to operate in a specific manner, so that the instructions stored in the computer-readable memory can form a manufactured product, including an implementation apparatus that implements a specified function. The computer program instructions can also be loaded onto a computer or other programmable data processing device, so that a series of operating steps are performed on the computer or other programmable device to produce a computer-implemented process, so that the instructions executed on a computer or other programmable data processing device can provide steps for implementing specified functions.

Although the principles herein have been shown in various embodiments, many modifications of structures, arrangements, proportions, elements, materials, and components particularly suitable for the specific environmental and operational requirements may be used without departing from the principles and scope of the present disclosure. The above modifications and other changes or amendments will be included in the scope of this disclosure.

The foregoing specific description has been described with reference to various embodiments. However, those skilled in the art will recognize that various modifications and changes can be made without departing from the scope of the present disclosure. Therefore, consideration of the present disclosure will be in an illustrative rather than a restrictive sense, and all such modifications will be included within the scope thereof. Also, the advantages of various embodiments, other advantages, and the solutions to problems have been described above. However, the benefits, advantages, solutions to problems, and any elements that can produce these, or solutions that make them more explicit, should not be interpreted as critical, necessary, or essential. The term "comprising", and any other variants thereof used herein are non-exclusive, so that the process, method, document, or device that includes a list of elements includes not only these elements, but also other elements that are not explicitly listed or do not belong to the process, method, system, document, or device. Furthermore, the term "coupling" and any other variations thereof used herein refer to physical connection, electrical connection, magnetic connection, optical connection, communicational connection, functional connection, and/or any other connection.

It should be noted that a person of ordinary skill in the art could also make several variations and improvements without departing from the concept of this disclosure, and these variations and improvements would all fall within the scope of protection of this disclosure. Therefore, the protection scope of this disclosure should be in accordance with the appended claims.

## Claims

1. A monitoring system, **characterized in that**, comprising a bedside monitor, a mobile monitoring device and a central station; wherein:
the bedside monitor is configured to monitor a patient and pair with the mobile monitoring device;
the mobile monitoring device is worn on a body of the patient, so as to monitor the patient for obtaining monitoring data; wherein a working mode of the mobile monitoring device comprises a continuous monitoring mode and an intermittent monitoring mode; wherein the mobile monitoring device is configured to:
when working in the intermittent monitoring mode; perform intermittent monitoring on the patient, receive, in a first wireless transmission manner, physiological parameter data of the patient, which data is measured by a spot measurement device, and transmit, to the central station in a second wireless transmission manner, monitoring data which is obtained during the intermittent monitoring and the physiological parameter data which is measured by the spot measurement device;
when working in the continuous monitoring mode; pair with the bedside monitor and jointly perform continuous monitoring on the patient with the bedside monitor, and transmit, to the central station and/or the paired bedside monitor in the second wireless transmission manner, monitoring data which is obtained during the continuous monitoring and physiological parameter data of the patient, which data is measured by a spot measurement device.

2. The monitoring system according to claim 1, **characterized in that**, comprising the spot measurement device, which is configured to measure at least one physiological parameter of the patient based on a user operation, so as to obtain the physiological parameter data, and is capable of enabling the mobile monitoring device to obtain the physiological parameter data in the first wireless transmission manner.

3. The monitoring system according to claim 1 or 2, **characterized in that**, in the monitoring system, a number of mobile monitoring devices is greater than a number of bedside monitor(s);
wherein some mobile monitoring device(s) respectively pair(s) with one bedside monitor and jointly work(s) with said bedside monitor when working, while some mobile monitoring device(s) do(es) not pair with any bedside monitor when working.

4. The monitoring system according to claim 1 or 2, **characterized in that**, in order to transmit, to the central station in the second wireless transmission manner, the physiological parameter data which is measured by the spot measurement device, the mobile monitoring device is further configured to:
transmit intermittently, to the central station in the second wireless transmission manner, the physiological parameter data which is measured by the spot measurement device, when working in the intermittent monitoring mode;
transmit in real time, to the central station in the second wireless transmission manner, the physiological parameter data which is measured by the spot measurement device, when working in the continuous monitoring mode.

5. The monitoring system according to claim 3, **characterized in that**, when a first mobile monitoring device pairs with a first bedside monitor and jointly works with the first bedside monitor, the first bedside monitor is further configured to disconnect from the first mobile monitoring device, so as to pair with a second mobile monitoring device, which is different from the first mobile monitoring device, and so as to jointly work with the second mobile monitoring device.

6. The monitoring system according to claim 1, **characterized in that**, when working in the continuous monitoring mode but not pairing with the bedside monitor, the mobile monitoring device is further configured to perform continuous monitoring on the patient alone, and transmit, to the central station in the second wireless transmission manner, monitoring data which is obtained during said continuous monitoring and the physiological parameter data of the patient, which data is measured by the spot measurement device.

7. The monitoring system according to any one of claims 1-6, **characterized in that**, the central station is configured to:
receive the physiological parameter data, which is measured by the spot measurement device and transmitted by the mobile monitoring device, and display, according to the patient, the received physiological parameter data; and receive the monitoring data, which is transmitted by the mobile monitoring device, process the received monitoring data, so as to obtain and display, according to the patient, at least one of physiological parameter value(s), parameter curve(s) and alarm event(s); and/or
determine whether the physiological parameter data exceeds a corresponding threshold, and if yes, output corresponding alarm information; and/or
determine whether one or more physiological parameters in the monitoring data exceed a corresponding threshold, and if yes, output corresponding alarm information.

8. The monitoring system according to any one of claims 1-7, **characterized in that**:
during a first preset period, the bedside monitor is configured to perform continuous monitoring on the patient;
after entering into a second preset period from the first preset period, the mobile monitoring device is further configured to work in the intermittent monitoring mode;
when entering into a third preset period under a condition that preset physiological parameter(s) in the monitoring data, which data is obtained in the intermittent monitoring mode, exceed(s) corresponding threshold(s), the mobile monitoring device is further configured to switch from the intermittent monitoring mode to the continuous monitoring mode.

9. The monitoring system according to claim 8, **characterized in that**, the mobile monitoring device is further configured to, under trigger(s) of a first preset condition and/or an instruction to switch the working mode, switch from the continuous monitoring mode to the intermittent monitoring mode;
wherein the first preset condition comprises: a duration, in which the mobile monitoring device works in the continuous monitoring mode, exceeds a preset time length; a preset time is reached; or the mobile monitoring device disconnects from the bedside monitor.

10. The monitoring system according to any one of claims 1-9, **characterized in that**:
when the mobile monitoring device does not pair with any bedside monitor and works alone in the intermittent monitoring mode, the central station is configured to: obtain a current EWS score of the patient; and control the mobile monitoring device to switch from the intermittent monitoring mode to the continuous monitoring mode, or output information, which indicates a user to pair the mobile monitoring device with one bedside monitor for working jointly and to use the continuous monitoring mode, when the EWS score is higher than a preset score; and/or
when the mobile monitoring device does not pair with any bedside monitor and works alone in the continuous monitoring mode, the central station is configured to: obtain a current EWS score of the patient; and control the mobile monitoring device to switch from the continuous monitoring mode to the intermittent monitoring mode, when the EWS score is lower than a preset score; and/or
when the mobile monitoring device pairs with the bedside monitor and jointly works with the bedside monitor in the continuous monitoring mode; the central station is configured to: obtain a current EWS score of the patient; and control the mobile monitoring device to switch from the continuous monitoring mode to the intermittent monitoring mode, and/or output information, which indicates a user to disconnect the mobile monitoring device from the paired bedside monitor, when the EWS score is lower than a preset score.

11. The monitoring system according to any one of claims 1-10, **characterized in that**, a measurement frequency for physiological parameter(s) of the mobile monitoring device in the continuous monitoring mode is higher than that in the intermittent monitoring mode; or
a frequency for transmitting monitoring data to the central station and/or the paired bedside monitor by the mobile monitoring device in the continuous monitoring mode is higher than that in the intermittent monitoring mode.

12. The monitoring system according to any one of claims 1-11, **characterized in that**, the bedside monitor is configured to measure at least one of a blood oxygen parameter, a blood pressure parameter, a pulse rate parameter, a body temperature parameter, an ECG parameter, a respiratory parameter, and a sleep parameter;
the physiological parameter data, which is measured by the spot measurement device, comprises at least one of: parameter data for body temperature, parameter data for blood pressure, and parameter data for blood sugar;
the monitoring data, which is obtained by the mobile monitoring device, comprises at least one of: parameter data for blood oxygen, parameter data for blood pressure, parameter data for pulse rate, parameter data for body temperature, ECG parameter data, respiratory parameter data, sleep parameter data, and exercise parameter data.

13. A monitoring system, **characterized in that**, comprising:
a central station;
a mobile monitoring device, which is worn on a body of a patient, so as to monitor the patient for obtaining monitoring data; wherein a working mode of the mobile monitoring device comprises a continuous monitoring mode and an intermittent monitoring mode; wherein the mobile monitoring device is configured to:
when working in the intermittent monitoring mode; perform intermittent monitoring on the patient, receive, in a first wireless transmission manner, physiological parameter data of the patient, which data is measured by a spot measurement device, and transmit, to the central station in a second wireless transmission manner, monitoring data which is obtained during the intermittent monitoring and the physiological parameter data which is measured by the spot measurement device;
when working in the continuous monitoring mode; perform continuous monitoring on the patient, and transmit, to the central station in the second wireless transmission manner, monitoring data which is obtained during the continuous monitoring and physiological parameter data of the patient, which data is measured by a spot measurement device.

14. The monitoring system according to claim 13, **characterized in that**, further comprising the spot measurement device, which is configured to measure at least one physiological parameter of the patient based on a user operation, so as to obtain the physiological parameter data, and is capable of enabling the mobile monitoring device to obtain the physiological parameter data in the first wireless transmission manner.

15. The monitoring system according to claim 13 or 14, **characterized in that**, in order to transmit, to the central station in the second wireless transmission manner, the physiological parameter data which is measured by the spot measurement device, the mobile monitoring device is further configured to:
transmit intermittently, to the central station in the second wireless transmission manner, the physiological parameter data which is measured by the spot measurement device, when working in the intermittent monitoring mode; or
transmit in real time, to the central station in the second wireless transmission manner, the physiological parameter data which is measured by the spot measurement device, when working in the continuous monitoring mode.

16. The monitoring system according to any one of claims 1-15, **characterized in that**, the first wireless transmission manner is short-range communication;
wherein in order to transmit, to the central station in the second wireless transmission manner, the physiological parameter data which is measured by the spot measurement device, the mobile monitoring device is further configured to:
associate the physiological parameter data which is measured by the spot measurement device with identification information of the patient; and
transmit, to the central station in the second wireless transmission manner, the physiological parameter data which is measured by the spot measurement device and the identification information of the patient.

17. The monitoring system according to any one of claims 1-15, **characterized in that**, the first wireless transmission manner is short-range communication, and the first wireless transmission manner is different from the second wireless transmission manner.

18. A central station, which is applicable in a monitoring system, wherein the monitoring system comprises a mobile monitoring device which is in wireless communicative connection with the central station; **characterized in that**:
the mobile monitoring device is worn on a body of a patient, so as to monitor the patient for obtaining monitoring data; wherein the mobile monitoring device is configured to receive, in a first wireless transmission manner, physiological parameter data of the patient, which data is measured by a spot measurement device; wherein a working mode of the mobile monitoring device comprises a continuous monitoring mode and an intermittent monitoring mode;
the central station is configured to:
receive, in a second wireless transmission manner, monitoring data which is obtained during intermittent monitoring and the physiological parameter data which is measured by the spot measurement device; wherein said monitoring data and the physiological parameter data are transmitted, when the mobile monitoring device works in the intermittent monitoring mode;
receive, in the second wireless transmission manner, monitoring data which is obtained during continuous monitoring and the physiological parameter data which is measured by the spot measurement device; wherein said monitoring data and the physiological parameter data are transmitted, when the mobile monitoring device works in the continuous monitoring mode;
display, according to the patient, the physiological parameter data which is measured by the spot measurement device; and
process the received monitoring data, so as to obtain and display, according to the patient, at least one of physiological parameter value(s), parameter curve(s) and alarm event(s).

19. The central station according to claim 18, **characterized in that**, the first wireless transmission manner is short-range communication;
wherein in order to transmit, to the central station in the second wireless transmission manner, the physiological parameter data which is measured by the spot measurement device, the mobile monitoring device is further configured to:
associate the physiological parameter data which is measured by the spot measurement device with identification information of the patient; and
transmit, to the central station in the second wireless transmission manner, the physiological parameter data which is measured by the spot measurement device and the identification information of the patient.

20. The central station according to claim 18, **characterized in that**, the central station is further configured to display an identifier of the spot measurement device or a measurement time of the physiological parameter data; when displaying, according to the patient, the physiological parameter data which is measured by the spot measurement device.

21. A monitoring system, **characterized in that**, comprising:
a bedside monitor;
a mobile monitoring device, which is worn on a body of a patient, so as to monitor the patient for obtaining monitoring data; wherein a working mode of the mobile monitoring device comprises a continuous monitoring mode and an intermittent monitoring mode; wherein the mobile monitoring device is configured to:
when working in the intermittent monitoring mode; perform intermittent monitoring on the patient, receive, in a first wireless transmission manner, physiological parameter data of the patient, which data is measured by a spot measurement device, and transmit, to the bedside monitor in a second wireless transmission manner, monitoring data which is obtained during the intermittent monitoring and the physiological parameter data which is measured by the spot measurement device;
when working in the continuous monitoring mode; pair with the bedside monitor and jointly perform continuous monitoring on the patient with the bedside monitor, and transmit, to the bedside monitor in the second wireless transmission manner, monitoring data which is obtained during the continuous monitoring and physiological parameter data of the patient, which data is measured by a spot measurement device.

22. The monitoring system according to claim 21, **characterized in that**, further comprising the spot measurement device, which is configured to measure at least one physiological parameter of the patient based on a user operation, so as to obtain the physiological parameter data, and is capable of enabling the mobile monitoring device to obtain the physiological parameter data in the first wireless transmission manner.

23. A monitoring system, comprising a mobile monitoring device and a spot measurement device; wherein:
the mobile monitoring device is worn on a body of a patient, so as to monitor the patient for obtaining first monitoring data; wherein the mobile monitoring device is in communicative connection with a target monitoring device;
the spot measurement device is configured to measure physiological parameter data of the patient; **characterized in that**:
the mobile monitoring device comprises a processor, a first wireless communication unit and a second wireless communication unit; wherein the mobile monitoring device is configured to receive, in a first wireless transmission manner through the first wireless communication unit, the physiological parameter data of the patient, which data is measured by the spot measurement device, and to transmit, to the target monitoring device in a second wireless transmission manner through the second wireless communication unit, the first monitoring data and the received physiological parameter data of the patient, which data is measured by the spot measurement device; wherein the first wireless communication unit and the second wireless communication unit use different wireless communication protocols, and the first wireless transmission manner is different from the second wireless transmission manner.

24. The monitoring system according to claim 23, **characterized in that**, the mobile monitoring device is further configured to receive, in the first wireless transmission manner through the first wireless communication unit, information which is related to the patient, and to transmit, to the target monitoring device in the second wireless transmission manner through the second wireless communication unit, the information which is related to the patient, the first monitoring data, and the received physiological parameter data of the patient, which data is measured by the spot measurement device.

25. The monitoring system according to claim 23 or 24, **characterized in that**, the processor is configured to:
obtain connection information from the target monitoring device in the first wireless transmission manner through the first wireless communication unit, or transmit connection information to the target monitoring device;
establish, based on the connection information, communicative connection with the target monitoring device in the second wireless transmission manner, through the second wireless communication unit; and
perform, through the second wireless communication unit, data interaction with the target monitoring device, based on the communicative connection.

26. The monitoring system according to any one of claims 23-25, **characterized in that**, the target monitoring device is a central station, a bedside monitor or another mobile monitoring device.

27. The monitoring system according to claim 23, **characterized in that**, a working mode of the mobile monitoring device comprises a continuous monitoring mode and an intermittent monitoring mode; wherein the mobile monitoring device is configured to:
when working in the intermittent monitoring mode; perform intermittent monitoring on the patient, receive, in the first wireless transmission manner through the first wireless communication unit, the physiological parameter data of the patient, which data is measured by the spot measurement device, and to transmit, to the target monitoring device in the second wireless transmission manner, monitoring data which is obtained during the intermittent monitoring and the physiological parameter data which is measured by the spot measurement device.

28. A spot measurement device which is applicable in a monitoring system; wherein the monitoring system comprises a mobile monitoring device, which is in wireless communicative connection with the spot measurement device, and a target monitoring device, which is in wireless communicative connection with the mobile monitoring device; wherein the mobile monitoring device is worn on a body of a patient, so as to monitor the patient for obtaining first monitoring data; wherein the mobile monitoring device is in communicative connection with the target monitoring device, so as to transmit the obtained first monitoring data to the target monitoring device, the spot measurement device is configured to measure physiological parameter data of the patient;
**characterized in that**, the spot measurement device comprises a processor and a first wireless communication unit; the spot measurement device is further configured to transmit, to the mobile monitoring device in a first wireless transmission manner through the first wireless communication unit, the physiological parameter data of the patient, which data is measured by the spot measurement device; so as to enable the mobile monitoring device to transmit, to the target monitor device in a second wireless transmission manner through a second wireless communication unit which is arranged at the mobile monitoring device, the received physiological parameter data of the patient, which data is measured by the spot measurement device; wherein the first wireless communication unit and the second wireless communication unit use different wireless communication protocols, and the first wireless transmission manner is different from the second wireless transmission manner.

29. The spot measurement device according to claim 28, **characterized in that**, the target monitoring device is a central station, a bedside monitor or another mobile monitoring device.

30. The spot measurement device according to claim 28, **characterized in that**, a working mode of the mobile monitoring device comprises a continuous monitoring mode and an intermittent monitoring mode;
wherein, when the mobile monitoring device works in the intermittent monitoring mode, the spot measurement device is further configured to transmit, to the mobile monitoring device in the first wireless transmission manner through the first wireless communication unit, the physiological parameter data of the patient, which data is measured by the spot measurement device; so as to enable the mobile monitoring device to transmit, to the target monitor device in the second wireless transmission manner through the second wireless communication unit which is arranged at the mobile monitoring device, the received physiological parameter data of the patient, which data is measured by the spot measurement device.

31. A monitoring method, **characterized in that**, comprising:
when a mobile monitoring device works in an intermittent monitoring mode; performing intermittent monitoring on a patient, receiving, in a first wireless transmission manner, physiological parameter data of the patient, which data is measured by a spot measurement device, and transmitting, to a central station in a second wireless transmission manner, monitoring data which is obtained during the intermittent monitoring and the physiological parameter data which is measured by the spot measurement device;
when a mobile monitoring device works in a continuous monitoring mode, pairing with a bedside monitor and jointly performing continuous monitoring on a patient with the bedside monitor, and transmitting, to a central station and/or the paired bedside monitor in the second wireless transmission manner, monitoring data which is obtained during the continuous monitoring and physiological parameter data which is measured by a spot measurement device.

32. The monitoring method according to claim 31, **characterized in that**, a number of the mobile monitoring devices is greater than a number of the bedside monitor(s);
wherein some mobile monitoring device(s) respectively pair(s) with one bedside monitor and jointly work(s) with said bedside monitor when working, while some mobile monitoring device(s) do(es) not pair with any bedside monitor when working.

33. The monitoring method according to claim 31, **characterized in that**, transmitting, to the central station in the second wireless transmission manner, the physiological parameter data which is measured by the spot measurement device, comprises:
transmitting intermittently, to the central station in the second wireless transmission manner, the physiological parameter data which is measured by the spot measurement device, in the intermittent monitoring mode;
transmitting in real time, to the central station in the second wireless transmission manner, the physiological parameter data which is measured by the spot measurement device, in the continuous monitoring mode.

34. The monitoring method according to claim 31, **characterized in that**, the first wireless transmission manner is short-range communication;
transmitting, to the central station in the second wireless transmission manner, the physiological parameter data which is measured by the spot measurement device, comprises:
associating the physiological parameter data which is measured by the spot measurement device with identification information of the patient; and
transmitting, to the central station in the second wireless transmission manner, the physiological parameter data which is measured by the spot measurement device and the identification information of the patient.

35. The monitoring method according to claim 32, **characterized in that**, when a first mobile monitoring device pairs with a first bedside monitor and jointly works with the first bedside monitor, the first bedside monitor is further configured to disconnect from the first mobile monitoring device, so as to pair with a second mobile monitoring device, which is different from the first mobile monitoring device, and so as to jointly work with the second mobile monitoring device.

36. The monitoring method according to claim 31, **characterized in that**, further comprising:
when the mobile monitoring device works in the continuous monitoring mode but does not pair with the bedside monitor; performing continuous monitoring on the patient alone, and transmitting, to the central station in the second wireless transmission manner, monitoring data which is obtained during said continuous monitoring and the physiological parameter data of the patient, which data is measured by the spot measurement device.

37. The monitoring method according to any one of claims 31-36, **characterized in that**, further comprising: by the central station,
receiving the physiological parameter data, which is measured by the spot measurement device and transmitted by the mobile monitoring device, and displaying, according to the patient, the received physiological parameter data; and receiving the monitoring data, which is transmitted by the mobile monitoring device, processing the received monitoring data, so as to obtain and display, according to the patient, at least one of physiological parameter value(s), parameter curve(s) and alarm event(s); and/or
determining whether the physiological parameter data exceeds a corresponding threshold, and if yes, outputting corresponding alarm information; and/or
determining whether one or more physiological parameters in the monitoring data exceed a corresponding threshold, and if yes, outputting corresponding alarm information.

38. The monitoring method according to any one of claims 31-37, **characterized in that**, further comprising:
during a first preset period, performing, through the bedside monitor, continuous monitoring on the patient;
after a second preset period is entered into from the first preset period, enabling the mobile monitoring device to work in the intermittent monitoring mode;
when a third preset period is entered into under a condition that preset physiological parameter(s) in the monitoring data, which data is obtained in the intermittent monitoring mode, exceed(s) corresponding threshold(s), switching the mobile monitoring device from the intermittent monitoring mode to the continuous monitoring mode.

39. The monitoring method according to claim 38, **characterized in that**, further comprising:
switching, under trigger(s) of a first preset condition and/or an instruction to switch the working mode, the mobile monitoring device from the continuous monitoring mode to the intermittent monitoring mode;
wherein the first preset condition comprises: a duration, in which the mobile monitoring device works in the continuous monitoring mode, exceeds a preset time length; a preset time is reached; or the mobile monitoring device disconnects from the bedside monitor.

40. The monitoring method according to any one of claims 31-39, **characterized in that**, further comprising:
when the mobile monitoring device does not pair with any bedside monitor and works alone in the intermittent monitoring mode; obtaining, by the central station, a current EWS score of the patient, and controlling, by the central station, the mobile monitoring device to switch from the intermittent monitoring mode to the continuous monitoring mode, or outputting, by the central station, information, which indicates a user to pair the mobile monitoring device with one bedside monitor for working jointly and to use the continuous monitoring mode, when the EWS score is higher than a preset score; and/or
when the mobile monitoring device does not pair with any bedside monitor and works alone in the continuous monitoring mode; obtaining, by the central station, a current EWS score of the patient, and controlling, by the central station, the mobile monitoring device to switch from the continuous monitoring mode to the intermittent monitoring mode, when the EWS score is lower than a preset score; and/or
when the mobile monitoring device pairs with the bedside monitor and jointly works with the bedside monitor in the continuous monitoring mode; obtaining, by the central station, a current EWS score of the patient, and controlling, by the central station, the mobile monitoring device switch from the continuous monitoring mode to the intermittent monitoring mode, and/or outputting, by the central station, information, which indicates a user to disconnect the mobile monitoring device from the paired bedside monitor, when the EWS score is lower than a preset score.

41. The monitoring method according to any one of claims 31-40, **characterized in that**, a measurement frequency for physiological parameter(s) of the mobile monitoring device in the continuous monitoring mode is higher than that in the intermittent monitoring mode; or
a frequency for transmitting monitoring data to the central station and/or the paired bedside monitor by the mobile monitoring device in the continuous monitoring mode is higher than that in the intermittent monitoring mode.

42. A monitoring method, **characterized in that**, comprising:
when a mobile monitoring device works in an intermittent monitoring mode; performing intermittent monitoring on a patient, receiving, in a first wireless transmission manner, physiological parameter data of the patient, which data is measured by a spot measurement device, and transmitting, to a central station in a second wireless transmission manner, monitoring data which is obtained during the intermittent monitoring and the physiological parameter data which is measured by the spot measurement device;
when a mobile monitoring device works in a continuous monitoring mode, performing continuous monitoring on a patient, and transmit, to a central station in the second wireless transmission manner, monitoring data which is obtained during the continuous monitoring and physiological parameter data of the patient, which data is measured by a spot measurement device.

43. A monitoring method, **characterized in that**, comprising:
when a mobile monitoring device works in an intermittent monitoring mode; performing intermittent monitoring on a patient, receiving, in a first wireless transmission manner, physiological parameter data of the patient, which data is measured by a spot measurement device, and transmitting, to a bedside monitor in a second wireless transmission manner, monitoring data which is obtained during the intermittent monitoring and the physiological parameter data which is measured by the spot measurement device;
when a mobile monitoring device works in a continuous monitoring mode, pairing with a bedside monitor and jointly performing continuous monitoring on a patient with the bedside monitor, and transmitting, to the paired bedside monitor in the second wireless transmission manner, monitoring data which is obtained during the continuous monitoring and physiological parameter data which is measured by a spot measurement device.

44. The monitoring method according to any one of claims 31-43, **characterized in that**, the first wireless transmission manner is short-range communication, and the first wireless transmission manner is different from the second wireless transmission manner.
